(19) Europäisches Patentamt
European Patent Office
Office européen des brevets

(11) EP 3 991 743 A1

(12) **EUROPEAN PATENT APPLICATION**
published in accordance with Art. 153(4) EPC

(43) Date of publication:
**04.05.2022 Bulletin 2022/18**

(21) Application number: **20846091.5**

(22) Date of filing: **07.07.2020**

(51) International Patent Classification (IPC):
***A61K 39/00*** *(2006.01)* ***A61K 31/7088*** *(2006.01)*
***A61P 37/04*** *(2006.01)* ***C07K 7/00*** *(2006.01)*

(52) Cooperative Patent Classification (CPC):
**A61K 31/7088; A61K 39/00; A61P 37/04;**
C07K 7/00

(86) International application number:
**PCT/JP2020/026560**

(87) International publication number:
**WO 2021/020047 (04.02.2021 Gazette 2021/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.07.2019 JP 2019139031**

(71) Applicant: **Juntendo Educational Foundation**
**Tokyo 113-8421 (JP)**

(72) Inventors:
- **MINAMINO Tohru**
  **Tokyo 1138421 (JP)**
- **NAKAGAMI Hironori**
  **Suita-shi, Osaka 565-0871 (JP)**

(74) Representative: **EP&C**
**P.O. Box 3241**
**2280 GE Rijswijk (NL)**

(54) **IMMUNE INDUCER AND PHARMACEUTICAL COMPOSITION FOR PREVENTING OR TREATING AGING-RELATED DISEASES**

(57) An immunity inducer contains, as an active ingredient, any of a peptide (a) or (b) below, or an expression vector having a structural gene including a region encoding the peptide. (a) A peptide including an amino acid sequence represented by SEQ ID NO: 1 or 2; (b) a peptide having immunity induction activity and including an amino acid sequence in which one or several amino acids are deleted, substituted or added in the amino acid sequence represented by SEQ ID NO: 1 or 2. A pharmaceutical composition for preventing or treating aging-related diseases includes the immunity inducer and an adjuvant.

FIG. 2A

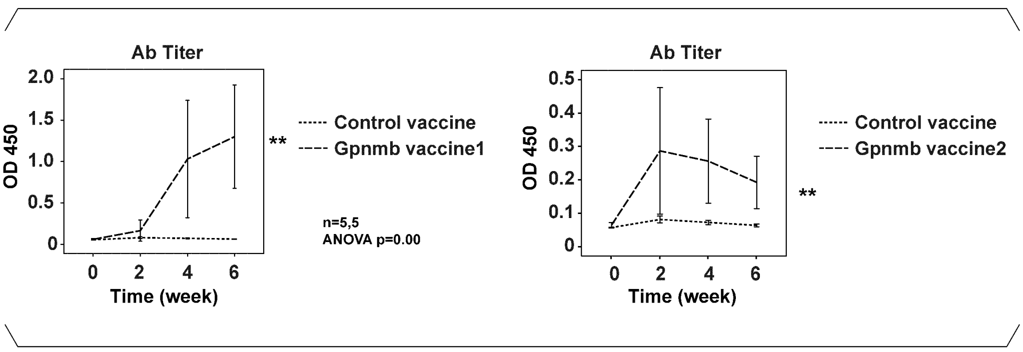


EP 3 991 743 A1

SEQUENCE LISTING

```
<110>  NIIGATA UNIVERSITY
       OSAKA UNIVERSITY

<120>  IMMUNE RESPONSE INDUCER

<130>  PC30268

<160>  22

<170>  PatentIn version 3.5

<210>  1
<211>  9
<212>  PRT
<213>  Artificial Sequence

<220>
<223>  Synthesized


<220>
<221>  MISC_FEATURE
<222>  (1)..(1)
<223>  Xaa indicates Arg or Lys.

<220>
<221>  MISC_FEATURE
<222>  (5)..(5)
<223>  Xaa indicates Met or Gly.

<400>  1

Xaa Arg Gly Asp Xaa Arg Trp Lys Asx
1               5


<210>  2
<211>  10
<212>  PRT
<213>  Artificial Sequence

<220>
<223>  Synthesized


<220>
<221>  MISC_FEATURE
<222>  (1)..(1)
<223>  Xaa indicates Arg or Gln.

<220>
<221>  MISC_FEATURE
<222>  (3)..(3)
<223>  Xaa indicates Gln or His.

<220>
<221>  MISC_FEATURE
<222>  (5)..(5)
<223>  Xaa indicates Leu or Asn.

<220>
```

```
<221>  MISC_FEATURE
<222>  (6)..(6)
<223>  Xaa indicates Arg or Val.

<220>
<221>  MISC_FEATURE
<222>  (10)..(6)
<223>  Xaa indicates Arg or Gly.

<220>
<221>  misc_feature
<222>  (10)..(10)
<223>  Xaa can be any naturally occurring amino acid

<400>  2

Xaa Ser Xaa His Xaa Xaa Phe Pro Asp Xaa
1               5                   10


<210>  3
<211>  9
<212>  PRT
<213>  Homo sapiens

<400>  3

Lys Arg Gly Asp Met Arg Trp Lys Asn
1               5


<210>  4
<211>  9
<212>  PRT
<213>  Mus musculus

<400>  4

Arg Arg Gly Asp Gly Arg Trp Lys Asp
1               5


<210>  5
<211>  10
<212>  PRT
<213>  Homo sapiens

<400>  5

Gln Ser His His Asn Val Phe Pro Asp Gly
1               5                   10


<210>  6
<211>  10
<212>  PRT
<213>  Mus musculus

<400>  6

Arg Ser Gln His Leu Arg Phe Pro Asp Arg
1               5                   10
```

<210> 7
<211> 572
<212> PRT
<213> Homo sapiens

<220>
<221> DOMAIN
<222> (63)..(71)

<220>
<221> DOMAIN
<222> (150)..(159)

<400> 7

```
Met Glu Cys Leu Tyr Tyr Phe Leu Gly Phe Leu Leu Leu Ala Ala Arg
1               5                   10                  15

Leu Pro Leu Asp Ala Ala Lys Arg Phe His Asp Val Leu Gly Asn Glu
            20                  25                  30

Arg Pro Ser Ala Tyr Met Arg Glu His Asn Gln Leu Asn Gly Trp Ser
        35                  40                  45

Ser Asp Glu Asn Asp Trp Asn Glu Lys Leu Tyr Pro Val Trp Lys Arg
    50                  55                  60

Gly Asp Met Arg Trp Lys Asn Ser Trp Lys Gly Gly Arg Val Gln Ala
65                  70                  75                  80

Val Leu Thr Ser Asp Ser Pro Ala Leu Val Gly Ser Asn Ile Thr Phe
                85                  90                  95

Ala Val Asn Leu Ile Phe Pro Arg Cys Gln Lys Glu Asp Ala Asn Gly
            100                 105                 110

Asn Ile Val Tyr Glu Lys Asn Cys Arg Asn Glu Ala Gly Leu Ser Ala
        115                 120                 125

Asp Pro Tyr Val Tyr Asn Trp Thr Ala Trp Ser Glu Asp Ser Asp Gly
    130                 135                 140

Glu Asn Gly Thr Gly Gln Ser His His Asn Val Phe Pro Asp Gly Lys
145                 150                 155                 160

Pro Phe Pro His His Pro Gly Trp Arg Arg Trp Asn Phe Ile Tyr Val
                165                 170                 175

Phe His Thr Leu Gly Gln Tyr Phe Gln Lys Leu Gly Arg Cys Ser Val
            180                 185                 190
```

Arg Val Ser Val Asn Thr Ala Asn Val Thr Leu Gly Pro Gln Leu Met
        195                 200                 205

Glu Val Thr Val Tyr Arg Arg His Gly Arg Ala Tyr Val Pro Ile Ala
    210                 215                 220

Gln Val Lys Asp Val Tyr Val Val Thr Asp Gln Ile Pro Val Phe Val
225                 230                 235                 240

Thr Met Phe Gln Lys Asn Asp Arg Asn Ser Ser Asp Glu Thr Phe Leu
                245                 250                 255

Lys Asp Leu Pro Ile Met Phe Asp Val Leu Ile His Asp Pro Ser His
            260                 265                 270

Phe Leu Asn Tyr Ser Thr Ile Asn Tyr Lys Trp Ser Phe Gly Asp Asn
        275                 280                 285

Thr Gly Leu Phe Val Ser Thr Asn His Thr Val Asn His Thr Tyr Val
    290                 295                 300

Leu Asn Gly Thr Phe Ser Leu Asn Leu Thr Val Lys Ala Ala Ala Pro
305                 310                 315                 320

Gly Pro Cys Pro Pro Pro Pro Pro Pro Pro Arg Pro Ser Lys Pro Thr
                325                 330                 335

Pro Ser Leu Ala Thr Thr Leu Lys Ser Tyr Asp Ser Asn Thr Pro Gly
            340                 345                 350

Pro Ala Gly Asp Asn Pro Leu Glu Leu Ser Arg Ile Pro Asp Glu Asn
        355                 360                 365

Cys Gln Ile Asn Arg Tyr Gly His Phe Gln Ala Thr Ile Thr Ile Val
    370                 375                 380

Glu Gly Ile Leu Glu Val Asn Ile Ile Gln Met Thr Asp Val Leu Met
385                 390                 395                 400

Pro Val Pro Trp Pro Glu Ser Ser Leu Ile Asp Phe Val Val Thr Cys
                405                 410                 415

Gln Gly Ser Ile Pro Thr Glu Val Cys Thr Ile Ile Ser Asp Pro Thr
            420                 425                 430

Cys Glu Ile Thr Gln Asn Thr Val Cys Ser Pro Val Asp Val Asp Glu

435 440 445

Met Cys Leu Leu Thr Val Arg Arg Thr Phe Asn Gly Ser Gly Thr Tyr
450 455 460

Cys Val Asn Leu Thr Leu Gly Asp Asp Thr Ser Leu Ala Leu Thr Ser
465 470 475 480

Thr Leu Ile Ser Val Pro Asp Arg Asp Pro Ala Ser Pro Leu Arg Met
485 490 495

Ala Asn Ser Ala Leu Ile Ser Val Gly Cys Leu Ala Ile Phe Val Thr
500 505 510

Val Ile Ser Leu Leu Val Tyr Lys Lys His Lys Glu Tyr Asn Pro Ile
515 520 525

Glu Asn Ser Pro Gly Asn Val Val Arg Ser Lys Gly Leu Ser Val Phe
530 535 540

Leu Asn Arg Ala Lys Ala Val Phe Phe Pro Gly Asn Gln Glu Lys Asp
545 550 555 560

Pro Leu Leu Lys Asn Gln Glu Phe Lys Gly Val Ser
565 570

<210> 8
<211> 574
<212> PRT
<213> Mus musculus

<220>
<221> DOMAIN
<222> (63)..(71)

<220>
<221> DOMAIN
<222> (150)..(159)

<400> 8

Met Glu Ser Leu Cys Gly Val Leu Gly Phe Leu Leu Leu Ala Ala Gly
1 5 10 15

Leu Pro Leu Gln Ala Ala Lys Arg Phe Arg Asp Val Leu Gly His Glu
20 25 30

Gln Tyr Pro Asp His Met Arg Glu His Asn Gln Leu Arg Gly Trp Ser
35 40 45

Ser Asp Glu Asn Glu Trp Asp Glu His Leu Tyr Pro Val Trp Arg Arg
    50                  55                  60

Gly Asp Gly Arg Trp Lys Asp Ser Trp Glu Gly Gly Arg Val Gln Ala
65                  70                  75                  80

Val Leu Thr Ser Asp Ser Pro Ala Leu Val Gly Ser Asn Ile Thr Phe
                85                  90                  95

Val Val Asn Leu Val Phe Pro Arg Cys Gln Lys Glu Asp Ala Asn Gly
            100                 105                 110

Asn Ile Val Tyr Glu Lys Asn Cys Arg Asn Asp Leu Gly Leu Thr Ser
        115                 120                 125

Asp Leu His Val Tyr Asn Trp Thr Ala Gly Ala Asp Asp Gly Asp Trp
    130                 135                 140

Glu Asp Gly Thr Ser Arg Ser Gln His Leu Arg Phe Pro Asp Arg Arg
145                 150                 155                 160

Pro Phe Pro Arg Pro His Gly Trp Lys Lys Trp Ser Phe Val Tyr Val
                165                 170                 175

Phe His Thr Leu Gly Gln Tyr Phe Gln Lys Leu Gly Arg Cys Ser Ala
            180                 185                 190

Arg Val Ser Ile Asn Thr Val Asn Leu Thr Ala Gly Pro Gln Val Met
        195                 200                 205

Glu Val Thr Val Phe Arg Arg Tyr Gly Arg Ala Tyr Ile Pro Ile Ser
    210                 215                 220

Lys Val Lys Asp Val Tyr Val Ile Thr Asp Gln Ile Pro Val Phe Val
225                 230                 235                 240

Thr Met Ser Gln Lys Asn Asp Arg Asn Leu Ser Asp Glu Ile Phe Leu
                245                 250                 255

Arg Asp Leu Pro Ile Val Phe Asp Val Leu Ile His Asp Pro Ser His
            260                 265                 270

Phe Leu Asn Asp Ser Ala Ile Ser Tyr Lys Trp Asn Phe Gly Asp Asn
        275                 280                 285

Thr Gly Leu Phe Val Ser Asn Asn His Thr Leu Asn His Thr Tyr Val
    290                 295                 300

```
Leu Asn Gly Thr Phe Asn Leu Asn Leu Thr Val Gln Thr Ala Val Pro
305                 310                 315                 320

Gly Pro Cys Pro Pro Pro Ser Pro Ser Thr Pro Pro Pro Pro Ser Thr
                325                 330                 335

Pro Pro Ser Pro Pro Pro Ser Pro Leu Pro Thr Leu Ser Thr Pro Ser
            340                 345                 350

Pro Ser Leu Met Pro Thr Gly Tyr Lys Ser Met Glu Leu Ser Asp Ile
        355                 360                 365

Ser Asn Glu Asn Cys Arg Ile Asn Arg Tyr Gly Tyr Phe Arg Ala Thr
    370                 375                 380

Ile Thr Ile Val Glu Gly Ile Leu Glu Val Ser Ile Met Gln Ile Ala
385                 390                 395                 400

Asp Val Pro Met Pro Thr Pro Gln Pro Ala Asn Ser Leu Met Asp Phe
                405                 410                 415

Thr Val Thr Cys Lys Gly Ala Thr Pro Met Glu Ala Cys Thr Ile Ile
            420                 425                 430

Ser Asp Pro Thr Cys Gln Ile Ala Gln Asn Arg Val Cys Ser Pro Val
        435                 440                 445

Ala Val Asp Gly Leu Cys Leu Leu Ser Val Arg Arg Ala Phe Asn Gly
    450                 455                 460

Ser Gly Thr Tyr Cys Val Asn Phe Thr Leu Gly Asp Asp Ala Ser Leu
465                 470                 475                 480

Ala Leu Thr Ser Thr Leu Ile Ser Ile Pro Gly Lys Asp Pro Asp Ser
                485                 490                 495

Pro Leu Arg Ala Val Asn Gly Val Leu Ile Ser Ile Gly Cys Leu Ala
            500                 505                 510

Val Leu Val Thr Met Val Thr Ile Leu Leu Tyr Lys Lys His Lys Ala
        515                 520                 525

Tyr Lys Pro Ile Gly Asn Cys Pro Arg Asn Thr Val Lys Gly Lys Gly
    530                 535                 540

Leu Ser Val Leu Leu Ser His Ala Lys Ala Pro Phe Phe Arg Gly Asp
545                 550                 555                 560
```

```
Gln Glu Lys Asp Pro Leu Leu Gln Asp Lys Pro Arg Thr Leu
                565                 570
```

```
<210>  9
<211>  1719
<212>  DNA
<213>  Homo sapiens

<400>  9
atggaatgtc tctactattt cctgggattt ctgctcctgg ctgcaagatt gccacttgat        60

gccgccaaac gatttcatga tgtgctgggc aatgaaagac cttctgctta catgagggag       120

cacaatcaat taaatggctg gtcttctgat gaaaatgact ggaatgaaaa actctaccca       180

gtgtggaagc ggggagacat gaggtggaaa aactcctgga agggaggccg tgtgcaggcg       240

gtcctgacca gtgactcacc agccctcgtg ggctcaaata taacatttgc ggtgaacctg       300

atattcccta gatgccaaaa ggaagatgcc aatggcaaca tagtctatga gaagaactgc       360

agaaatgagg ctggtttatc tgctgatccg tatgtttaca actggacagc atggtcagag       420

gacagtgacg gggaaaatgg caccggccaa agccatcata acgtcttccc tgatgggaaa       480

ccttttcctc accaccccgg atggagaaga tggaatttca tctacgtctt ccacacactt       540

ggtcagtatt tccagaaatt gggacgatgt tcagtgagag tttctgtgaa cacagccaat       600

gtgacacttg ggcctcaact catggaagtg actgtctaca gaagacatgg acgggcatat       660

gttcccatcg cacaagtgaa agatgtgtac gtggtaacag atcagattcc tgtgtttgtg       720

actatgttcc agaagaacga tcgaaattca tccgacgaaa ccttcctcaa agatctcccc       780

attatgtttg atgtcctgat tcatgatcct agccacttcc tcaattattc taccattaac       840

tacaagtgga gcttcgggga taatactggc ctgtttgttt ccaccaatca tactgtgaat       900

cacacgtatg tgctcaatgg aaccttcagc cttaacctca ctgtgaaagc tgcagcacca       960

ggaccttgtc cgccaccgcc accaccaccc agaccttcaa aacccacccc ttctttagca      1020

actactctaa aatcttatga ttcaaacacc ccaggacctg ctggtgacaa ccccctggag      1080

ctgagtagga ttcctgatga aaactgccag attaacagat atggccactt tcaagccacc      1140

atcacaattg tagagggaat cttagaggtt aacatcatcc agatgacaga cgtcctgatg      1200

ccggtgccat ggcctgaaag ctccctaata gactttgtcg tgacctgcca agggagcatt      1260

cccacggagg tctgtaccat catttctgac cccacctgcg agatcaccca gaacacagtc      1320

tgcagccctg tggatgtgga tgagatgtgt ctgctgactg tgagacgaac cttcaatggg      1380

tctgggacgt actgtgtgaa cctcaccctg ggggatgaca caagcctggc tctcacgagc      1440

accctgattt ctgttcctga cagagaccca gcctcgcctt taaggatggc aaacagtgcc      1500

ctgatctccg ttggctgctt ggccatattt gtcactgtga tctccctctt ggtgtacaaa      1560
```

aaacacaagg aatacaaccc aatagaaaat agtcctggga atgtggtcag aagcaaaggc 1620

ctgagtgtct ttctcaaccg tgcaaaagcc gtgttcttcc cgggaaacca ggaaaaggat 1680

ccgctactca aaaaccaaga atttaaagga gtttcttaa 1719

<210> 10
<211> 1725
<212> DNA
<213> Mus musculus

<400> 10
atggaaagtc tctgcggggt cctgggattt ctgctgctgg ctgcaggact gcctctccag 60

gctgccaagc gatttcgtga tgtgctgggc catgaacagt atcccgatca catgagagag 120

cacaaccaat tacgtggctg gtcttcggat gaaaatgaat gggatgaaca cctgtatcca 180

gtgtggagga ggggagacgg caggtggaag gactcctggg aaggaggccg tgtgcaggca 240

gtcctgacca gtgactcacc ggctctggtg ggttccaata tcacttttgt ggtgaacctg 300

gtgttcccca gatgccagaa ggaagatgct aatggcaata tcgtctatga gaagaactgc 360

aggaatgatt tgggactgac atctgacctg catgtctaca actggactgc aggggcagat 420

gatggtgact gggaagatgg caccagccga agccagcatc tcaggttccc ggacaggagg 480

cccttccctc gcccccatgg atggaagaaa tggagctttg tctacgtctt tcacacactt 540

ggccagtatt tccaaaaact gggtcggtgt tcagcacggg tttctataaa cacagtcaac 600

ttgacagctg gccctcaggt catggaagtg actgtctttc gaagatacgg ccgggcatac 660

attcccatct cgaaggtgaa agatgtgtat gtgataacag atcagatccc tgtattcgtg 720

accatgtccc agaagaatga caggaacttg tctgatgaga tcttcctcag agacctcccc 780

atcgtcttcg atgtcctcat tcatgatccc agccacttcc tcaacgactc tgccatttcc 840

tacaagtgga actttgggga caacactggc ctgtttgtct ccaacaatca cactttgaat 900

cacacttatg tgctcaatgg aaccttcaac cttaacctca ccgtgcaaac tgcagtgccc 960

gggccatgcc ctcccccttc gccttcgact ccgcctccac cttcaactcc gccctcacct 1020

ccgccctcac ctctgcccac attatcaaca cctagcccct ctttaatgcc tactggttac 1080

aaatccatgg agctgagtga catttccaat gaaaactgcc gaataaacag atatggctac 1140

ttcagagcca ccatcacaat tgtagagggg atcctggaag tcagcatcat gcagatagca 1200

gatgtcccca tgcccacacc gcagcctgcc aactccctga tggacttcac tgtgacctgc 1260

aaagggggcca cccccatgga agcctgtacg atcatctccg accccacctg ccagatcgcc 1320

cagaaccggg tctgcagccc tgtggctgtg gatgggctgt gcctgctgtc tgtgagaaga 1380

gccttcaatg ggtctggcac ctactgtgtg aatttcactc tgggagatga tgcaagcctg 1440

gccctcacca gcaccctgat ctctatccct ggcaaagacc cagactcccc tctgagagca 1500

gtgaatggtg tcctgatctc catcggctgc ctggctgtgc ttgtcaccat ggttaccatc 1560

ttgctgtaca aaaaacacaa ggcgtacaag ccaataggaa actgccccag gaacacggtc 1620

aagggcaagg gcctgagtgt tctcctcagt cacgcgaaag ccccgttctt ccgaggagac 1680

caggagaagg atccattgct ccaggacaag ccaaggacac tctaa 1725

<210> 11
<211> 27
<212> DNA
<213> Homo sapiens

<400> 11
aagcggggag acatgaggtg gaaaaac 27

<210> 12
<211> 27
<212> DNA
<213> Mus musculus

<400> 12
aggaggggag acggcaggtg gaaggac 27

<210> 13
<211> 30
<212> DNA
<213> Homo sapiens

<400> 13
caaagccatc ataacgtctt ccctgatggg 30

<210> 14
<211> 30
<212> DNA
<213> Mus musculus

<400> 14
cgaagccagc atctcaggtt cccggacagg 30

<210> 15
<211> 18
<212> DNA
<213> Artificial Sequence

<220>
<223> Synthesized

<400> 15
acggcaggtg gaaggact 18

<210> 16
<211> 19
<212> DNA
<213> Artificial Sequence

<220>
<223> Synthesized

<400> 16
cggtgagtca ctggtcagg 19

<210> 17
<211> 20
<212> DNA
<213> Artificial Sequence

<220>
<223> Synthesized

<400> 17
tccacagcga tatccagaca 20

<210> 18
<211> 20
<212> DNA
<213> Artificial Sequence

<220>
<223> Synthesized

<400> 18
ggacatcacc aggattggac 20

<210> 19
<211> 21
<212> DNA
<213> Artificial Sequence

<220>
<223> Synthesized

<400> 19
gggttttctt ggtgaagttc g 21

<210> 20
<211> 19
<212> DNA
<213> Artificial Sequence

<220>
<223> Synthesized

<400> 20
ttgcccatca tcatcacct 19

<210> 21
<211> 18
<212> DNA
<213> Artificial Sequence

<220>
<223> Synthesized

```
<400>  21
gatgcccagg gaagacag                                                18


<210>  22
<211>  21
<212>  DNA
<213>  Artificial Sequence

<220>
<223>  Synthesized

<400>  22
acaatgaagc attttggata a                                            21
```

## Description

### TECHNICAL FIELD

The present invention relates to an immunity inducer and a pharmaceutical composition for preventing or treating aging-related diseases. Priority is claimed on Japanese Patent Application No. 2019-139031, filed July 29, 2019, the content of which is incorporated herein by reference.

### BACKGROUND ART

Accumulation of aging cells in various tissues has been reported to have a pathological role in age-related diseases. Direct inhibition of aging regulating factors of cells (for example, p53, p21, and the like) prevents the development of age-related pathologies, but has a risk of increasing the incidence of cancer. Recently, it has been reported that removal of aging cells (senolysis) reversibly improves the pathological aging phenotype of aged mice without leading to the onset of cancer. The inventors identified transmembrane glycoprotein nonmetastatic melanoma protein B (GPNMB) protein as an aging cell-specific molecule with a transmembrane domain based on analysis of transcriptome data from aged vascular endothelial cells, and clarified that the expression of GPNMB protein was up-regulated in vascular cells of patients and mice with atherosclerosis (for example, refer to Patent Document 1).
On the other hand, it has been reported that GPNMB protein is overexpressed in most human malignant melanoma, breast cancer, and brain tumors, and an immunity inducer containing a peptide fragment of GPNMB protein as an active ingredient (for example, refer to Patent Document 2) or an antibody that is specifically bonded to GPNMB protein (for example, refer to Patent Document 3), for the purpose of treating or preventing cancer by immunotherapy, has been developed.
However, epitopes of GPNMB protein that are effective for the purpose of removing aging cells are not known.

### PRIOR ART LITERATURE

#### Patent Document

Patent Document 1: Japanese Unexamined Patent Application, First Publication No. 2017-112849
Patent Document 2: PCT International Publication No. WO2009/054470
Patent Document 3: Published Japanese Translation No. 2008-521411 of the PCT International Publication

### DISCLOSURE OF INVENTION

#### Problems to be Solved by the Invention

The present invention has been made in view of the above circumstances, and provides an immunity inducer effective for preventing or treating aging-related diseases and a pharmaceutical composition for preventing or treating aging-related diseases containing the immunity inducer.

#### Means for Solving the Problems

As a result of intensive research to achieve the above object, the inventors have found that regions at positions 63 to 71 or positions 150 to 159 in the amino acid sequence of human GPNMB protein are effective epitopes for removing aging cells, and that aging cells can be removed and the phenotype associated with cellular aging can be improved by administering a peptide including the amino acid sequence of the region, thereby completing the present invention.
That is, the present invention includes the following aspects.
The immunity inducer according to a first aspect of the present invention includes, as an active ingredient, any of the peptide (a) or (b) below, or an expression vector having a structural gene including a region encoding the peptide.

(a) a peptide including an amino acid sequence represented by SEQ ID NO: 1 or 2;
(b) a peptide having immunity induction activity and including an amino acid sequence in which one or several amino acids are deleted, substituted, or added in the amino acid sequence represented by SEQ ID NO: 1 or 2.

The peptide may include the amino acid sequence represented by any of SEQ ID NOS: 3 to 6.

The immunity inducer according to the first aspect may include the peptide as an active ingredient.
In the immunity inducer according to the first aspect, a carrier protein may be further bonded to the peptide.
The immunity inducer according to the first aspect may be used for removing aging cells.
A pharmaceutical composition for preventing or treating aging-related diseases according to a second aspect of the present invention includes an immunity inducer and an adjuvant.

Effects of the Invention

The immunity inducer of the aspect is effective for preventing or treating aging-related diseases.

BRIEF DESCRIPTION OF THE DRAWINGS

Fig. 1A is a diagram showing a configuration of a construct in Reference Example 1.
Fig. 1B is an image showing a detection result of Gpnmb-dependent luciferase activity in Reference Example 1.
Fig. 1C is an image showing a result of aging-related acidic β-galactosidase (SA-β-gal) staining in Reference Example 1. The scale bar indicates 5 mm.
Fig. 1D is a graph quantifying a result of SA-β-gal staining in Reference Example 1.
Fig. 1E is a graph showing a relative RNA expression level of Gpnmb, Cdknla, and Cdkn2a in Reference Example 1.
Fig. IF is a graph showing results of a glucose tolerance test and an insulin tolerance test in Reference Example 1.
Fig. 1G is an image showing a detection result of arteriosclerotic plaque in Reference Example 1.
Fig. 1H is a graph quantifying a detection result of arteriosclerotic plaque in Reference Example 1.
Fig. 2A is a graph showing antibody titers in mice administered with peptide vaccine 1, peptide vaccine 2, or control vaccine in Example 1.
Fig. 2B is an image showing a detection result of Gpnmb-dependent luciferase activity in Example 1.
Fig. 2C is a graph showing a relative RNA expression level of Gpnmb, Cdknla, and Cdkn2a in Example 1.
Fig. 2D is an image showing a result of Western blotting analysis in Example 1.
Fig. 2E is a graph showing a result of Antibody-Dependent-Cellular-Cytotoxicity (ADCC) activity analysis in Example 1.
Fig. 2F is an image showing a detection result of $p19^{Arf}$-dependent luciferase activity in Example 1.
Fig. 2G is an image showing a result of SA-β-gal staining in Example 1. The scale bar indicates 5 mm.
Fig. 2H is a graph quantifying a result of SA-β-gal staining in Example 1.
Fig. 2I is a graph showing results of a glucose tolerance test and an insulin tolerance test in Example 1. The upper two graphs are the results of administration of peptide vaccine 1, and the lower two graphs are the results of administration of peptide vaccine 2.
Fig. 2J is an image (top) showing a detection result of arteriosclerotic plaque in Example 1 and an image (bottom) showing a detection result of Gpnmb-dependent luciferase activity in an artery.
Fig. 2K is a graph quantifying a detection result of arteriosclerotic plaque in Example 1.
Fig. 2L is a graph showing a result of an open field test in Example 1.
Fig. 2M is a graph showing a survival curve of Zmpste24-deficient mice in Example 1.

EMBODIMENTS FOR CARRYING OUT THE INVENTION

Hereinafter, the immunity inducer according to the embodiment of the present invention will be described in detail.

<<Immunity inducer>>

An immunity inducer of the present embodiment includes, as an active ingredient, a peptide including regions at positions 63 to 71 or positions 150 to 159 in an amino acid sequence of a human or mouse GPNMB protein. Such an immunity inducer is also referred to as "peptide vaccine".
In addition, in the present specification, "includes as an active ingredient" means including a therapeutically effective amount of a peptide or an expression vector having a structural gene including a region encoding the peptide.
GPNMB is a one-time transmembrane protein and is known to be partially cleaved and secreted into the blood.
As shown in Examples described later, the inventors have clarified that aging cells can be removed by administering a peptide including regions at positions 63 to 71 or positions 150 to 159 in the amino acid sequence of the mouse GPNMB protein. In addition, GPNMB protein is highly conserved in all mammals. Therefore, based on such findings, a peptide including regions corresponding to the regions at positions 63 to 71 or positions 150 to 159 in the amino acid sequence of the mouse GPNMB protein can be used as a prophylactic or therapeutic agent for aging-related diseases.

The RefSeq accession number of the human GPNMB protein is NP_001005340. In addition, the RefSeq accession number of the mouse GPNMB protein is NP_444340. The amino acid sequence of the human GPNMB protein is shown in SEQ ID NO: 7. The amino acid sequence of the mouse GPNMB protein is shown in SEQ ID NO: 8. Regions corresponding to the regions at positions 63 to 71 or positions 150 to 159 in the amino acid sequence of the mouse GPNMB protein of a mammal other than mouse can be examined using a known multi-alignment tool.

In addition, in the present specification, "aging cell" means a cell which shows an increase in the expression amount of an aging marker as compared with a normal cell. Examples of the aging marker include aging-related acidic β-galactosidase, P53, $P16^{INK4a}$, $P21^{CIP1}$, and the like. It is known that aging cells are characterized by irreversible cessation of growth in the G1 phase and are formed by the involvement of suppression of genes that promote cell cycle progression and increased expression of P53, $P16^{INK4a}$, and $P21^{CIP1}$ that inhibit the cell cycle.

Aging cells can be cells that have stopped dividing but remain metabolically active. Non-dividing cells can survive for weeks, but are unable to grow and replicate DNA despite the presence of sufficient space, nutrients, and growth factors in a medium. Therefore, this cessation of division is essentially permanent, as aging cells cannot be stimulated to grow even if physiological stimuli are applied to the aging cells.

Aging cells can differ from non-aging cells in one or more of the following: 1) Aging cells cease to grow and cannot be stimulated to re-enter the cell cycle with physiological mitogen; 2) aging cells become resistant to apoptotic cell death; and 3) aging cells acquire altered differentiation function.

Aging cells can be caused by replicative cellular aging, precocious cellular aging, treatment-induced cellular aging, or the like. Aging cells caused by replicative cellular aging may undergo cell division a plurality of times, for example, 40 times or more, 50 times or more, 60 times or more, 70 times or more, and 80 times or more. Aging cells caused by precocious cellular aging are not limited, but can be induced by ultraviolet rays, reactive oxygen species, environmental toxins, smoking, ionizing radiation, chromatin structural distortions, excessive mitogen signaling, and the like. In specific embodiments, precocious cellular aging can be induced by ionizing radiation. In another specific embodiment, precocious cellular aging can be induced by ectopic transfection using Ras protein. Aging cells caused by treatment-induced cellular aging can be induced by radiation therapy, chemotherapy, DNA damage therapy, and the like.

Aging cells that are subjects of the present invention can generally be eukaryotic cells. Aging cells include, but are not limited to: breast epithelial cells, keratinocytes, myocardial cells, cartilage cells, endothelial cells (great vessel), endothelial cells (tiny vessel), epithelial cells, fibroblasts, hair papilla cells, hepatocytes, melanin cells, osteoblasts, adipose precursor cells, immune system cells, skeletal muscle cells, smooth muscle cells, adipose cells, neurons, glia cells, contractile cells, exocrine epithelial cells, extracellular matrix cells, hormone-secreting cells, keratinized epithelial cells, pancreatic islet cells, crystalline cells, mesenchymal stem cells, pancreatic adenocarcinoma cells, small intestinal Paneth cells, hematopoietic cells, neural cells, cells that support sensory organs and peripheral nerve cells, and wet multilayer barrier epithelial cells.

In addition, aging cells that are subjects of the present invention can also be found in regenerative tissues including a vascular system, a hematopoietic system, epithelial organs, and stroma. In addition, aging cells can also be found at aged sites or sites of chronic conditions associated with aging (for example, osteoarthritis and atherosclerosis). In addition, aging cells can be associated with benign dysplasia lesions and benign prostate hyperplasia. In an embodiment, aging cells can be found in normal tissues after DNA damage therapy. In another specific embodiment, aging cells can be found at a site of pathology associated with aging.

The number of aging cells in various organs or tissues usually increases with age. Accumulation of aging cells can further accelerate deterioration under aging and aging-related diseases. For example, the accumulation of aging cells in aging tissues can contribute to age-related tissue dysfunction, diminished regenerative capacity, and diseases. In an embodiment, aging tissues in which aging cells are accumulated lack an ability to respond to stresses that require growth, thereby resulting in the reduced health seen with aging.

In addition, in the present specification, "removal of aging cells" means removing aging cells from tissues or organs, or killing aging cells. It is particularly preferable that cells that are not aging cells (hereinafter, referred to as "non-aging cells") not be significantly killed, and that aging cells be selectively or specifically killed.

In addition, in the present specification, "aging-related diseases" include any diseases or conditions that are wholly or partially mediated by the induction or maintenance of non-proliferative or aging conditions in cells or cell populations in a subject. The aging-related diseases include tissue or organ decline in which signs of the conditions are not visible, or visible conditions such as degenerative diseases or impaired dysfunction.

Examples of the aging-related diseases include Alzheimer's disease, Parkinson's disease, cataract, luteal degeneration, glaucoma, atherosclerosis, acute coronary syndrome, myocardial infarction, heart failure, diabetes, stroke, hypertension, idiopathic pulmonary fibrosis (IPF), chronic obstructive pulmonary disease (COPD), osteoarthritis, coronary artery disease, cerebrovascular disease, periodontal disease, atrophy or fibrosis in various tissues, brain or heart damage, treatment-related myelodysplastic syndrome, and the like. In addition, the aging-related diseases include Hutchinson-Gilford-Progeria syndrome, Werner syndrome, Cockayne syndrome, xeroderma pigmentosum, telangiectasia cerebral ataxia, Fanconi anemia, neuropathy anemia, and the like.

Further examples of aging-related diseases include cardiovascular diseases such as angina, circulatory disease such as aortic aneurysm, arrhythmia, cerebral aneurysm, cardiac diastolic dysfunction, cardiac fibrosis, myocardial disease, carotid artery disease, coronary artery thrombosis, endocarditis, hypercholesterolemia, hyperlipidemia, mitral valve prolapse, peripheral vascular disease; inflammatory or autoimmune disease such as herniated disc, oral mucositis, erythema, interstitial cystitis, scleroderma, and alopecia; neurodegenerative disease such as dementia, Huntington's disease, motor and neurological dysfunction, memory deterioration associated with aging, depression, and mood disorders; metabolic disease such as metabolic syndrome; lung disease such as deterioration of lung function associated with aging, asthma, bronchial dilatation, cystic fibrosis, and emphysema; gastrointestinal disease such as Barrett's esophagus; disease associated with aging such as liver fibrosis, muscle fatigue, oral mucosal fibrosis, pancreatic fibrosis, benign prostate hyperplasia (BPH), and sleep disorder; reproductive disorder such as menopause, decreased egg supply, decreased sperm viability, decreased fertility, decreased libido, decreased erectile function, and excitement; skin disease such as atopic dermatitis, cutaneous erythema, cutaneous lymphoma, dysesthesia, eczema, eosinophil dermatitis, fibrous growth of skin, pigmentation, immune vesicular disease, mother's plaque, asthma vulgaris, pruritus, psoriasis, rash, reactive neutrophil skin disease, wrinkles, and urticaria; post-transplant kidney fibrosis; carotid artery thrombosis, and the like.

In addition, preferred examples of aging-related diseases include circulatory disease such as heart failure, arteriosclerosis, myocardial infarction, diabetes, arteriosclerotic cerebrocardiovascular disease, and hypertension; cerebrovascular disease such as cerebral infarction and cerebral hemorrhage; metabolic disease such as hyperlipidemia; respiratory disease such as pulmonary fibrosis and pulmonary emphysema; motor syndrome such as skeletal muscle atrophy (sarcopenia) and osteoarthritis; geriatric syndrome such as dementia and frail; chronic kidney disease; eye disease such as cataract, glaucoma, age-related yellow spot degeneration, and aging eyes; age-related hair loss; age-related hearing loss; age-related pain such as lower back pain and joint pain; skin disease such as sebum-deficient eczema and skin pruritus; liver disease such as fatty liver, non-alcoholic steatosis (NASH), and liver cirrhosis; bone disease such as osteoporosis and osteoarthritis; premature senility such as Hutchinson-Gilford-Progeria syndrome, Werner syndrome, Cockayne syndrome, and Rothmund-Thomson syndrome; and the like.

Accumulation of aging cells is known to promote pathological conditions such as aging-related diseases. Therefore, it is possible to prevent or treat a disease that is expected to show an improved pathological condition by administering the immunity inducer of the present embodiment to activate the immune system in the body and remove aging cells.

In addition, as shown in Examples described later, the inventors clarified that arteriosclerotic plaque is reduced by administering a peptide including regions at positions 63 to 71 or positions 150 to 159 in the amino acid sequence of the mouse GPNMB protein. Therefore, the peptide is particularly suitable as a prophylactic or therapeutic agent for atherosclerosis.

Specifically, the immunity inducer of the present embodiment contains the peptide (a) below as an active ingredient.

(a) A peptide including the amino acid sequence represented by SEQ ID NO: 1 or 2.

The amino acid sequence represented by SEQ ID NO: 1 in (a) is the amino acid sequence at positions 63 to 71 in the amino acid sequence of the human or mouse GPNMB protein. The amino acid sequence represented by SEQ ID NO: 2 is the amino acid sequence at positions 150 to 159 in the amino acid sequence of the human or mouse GPNMB protein.

SEQ ID NO: 1: XRGDXRWKB
SEQ ID NO: 2: XSXHXXFPDX

In SEQ ID NO: 1, X positioned first from the left is arginine or lysine, X positioned fifth from the left is glycine or methionine, and B positioned ninth from the left is aspartic acid or asparagine. That is, the amino acid sequence represented by SEQ ID NO: 1 is the following amino acid sequence. In addition, SEQ ID NO: 3 is the amino acid sequence of the regions at positions 63 to 71 in the amino acid sequence of the human GPNMB protein. SEQ ID NO: 4 is the amino acid sequence of the regions at positions 63 to 71 in the amino acid sequence of the mouse GPNMB protein.

SEQ ID NO: 3: KRGDMRWKN
SEQ ID NO: 4: RRGDGRWKD

In addition, X positioned first from the left in SEQ ID NO: 2 is arginine or glutamine, X positioned third from the left is glutamine or histidine, X positioned fifth from the left is leucine or asparagine, X positioned sixth from the left is arginine or valine, and X positioned tenth from the left is arginine or glycine. That is, the amino acid sequence represented by SEQ ID NO: 2 is the following amino acid sequence. SEQ ID NO: 5 is the amino acid sequence of the regions at positions 150 to 159 in the amino acid sequence of the human GPNMB protein. SEQ ID NO: 6 is the amino acid sequence of the regions at positions 150 to 159 in the amino acid sequence of the mouse GPNMB protein.

SEQ ID NO: 5: QSHHNVFPDG
SEQ ID NO: 6: RSQHLRFPDR

Among these, it is preferable that the peptide contained in the immunity inducer of the present embodiment be a peptide including the amino acid sequence represented by any of SEQ ID NOS: 3 to 6.

The immunity inducer of the present embodiment may contain the peptide (b) below as a peptide functionally equivalent to the peptide (a).

(b) a peptide having immunity induction activity and including an amino acid sequence in which one or several amino acids are deleted, substituted, or added in the amino acid sequence represented by SEQ ID NO: 1 or 2.

Here, the number of amino acids that may be deleted, substituted, or added is preferably 1 or more and 2 or less, and more preferably 1.

In the amino acid sequence represented by SEQ ID NO: 1, examples of the amino acid residues that may be deleted preferably include X (amino acid residue of N-terminal) positioned first from the left in SEQ ID NO: 1 or B (amino acid residue of C-terminal) positioned ninth from the left. In the amino acid sequence represented by SEQ ID NO: 1, any amino acid residue may be added to the N-terminal or C-terminal, but as the amino acid residue added to the N-terminal or C-terminal, other amino acid residues having a chemically similar side chain to the amino acid residue positioned at the N-terminal or C-terminal of the amino acid sequence represented by SEQ ID NO: 1 are preferably added. Other amino acid residues having a chemically similar side chain will be described later.

In the amino acid sequence represented by SEQ ID NO: 1, examples of the amino acid residue that may be substituted preferably include X positioned first from the left, X positioned fifth from the left, or B positioned ninth from the left in SEQ ID NO: 1. These amino acid residues may be substituted with any amino acid residue, but are preferably substituted with other amino acid residues having a chemically similar side chain to the amino acid residue to be substituted. Other amino acid residues having a chemically similar side chain will be described later.

In addition, in the amino acid sequence represented by SEQ ID NO: 2, examples of the amino acid residues that may be deleted preferably include X (amino acid residue of N-terminal) positioned first from the left or X (amino acid residue of C-terminal) positioned the tenth from the left in SEQ ID NO: 2. In the amino acid sequence represented by SEQ ID NO: 2, any amino acid residue may be added to the N-terminal or C-terminal, but the amino acid residues added to the N-terminal or C-terminal preferably include other amino acid residues having a chemically similar side chain to the amino acid residue positioned at the N-terminal or C-terminal of the amino acid sequence represented by SEQ ID NO: 2. Other amino acid residues having a chemically similar side chain will be described later.

In the amino acid sequence represented by SEQ ID NO: 2, the amino acid residues that may be substituted preferably include X positioned first from the left, X positioned third from the left, X positioned fifth from the left, and X positioned the sixth from the left, or X positioned the tenth from the left. These amino acid residues may be substituted with any amino acid residue, but are preferably substituted with other amino acid residues having a chemically similar side chain to the amino acid residue to be substituted. Other amino acid residues having a chemically similar side chain will be described later.

In addition, in the present specification, "substitution" means substitution with other amino acid residues having a chemically similar side chain. A group of amino acid residues having a chemically similar amino acid side chain is well known in the technical field to which the peptide contained in the immunity inducer of the present embodiment belongs. For example, acidic amino acids (aspartic acid and glutamic acid), basic amino acids (lysine, arginine, and histidine), and neutral amino acids are classified into amino acids having a hydrocarbon chain (glycine, alanine, valine, leucine, isoleucine, and proline), amino acids having a hydroxy group (serine and threonine), amino acids containing sulfur (cysteine and methionine), amino acids having an amide group (asparagine and glutamine), amino acids having an imino group (proline), amino acids having an aromatic group (phenylalanine, tyrosine, and tryptophan), and the like. Examples of commonly occurring amino acid substitutions include alanine/serine, valine/isoleucine, aspartic acid/glutamic acid, threonine/serine, alanine/glycine, alanine/threonine, serine/asparagine, alanine/valine, serine/glycine, tyrosine/phenylalanine, alanine/proline, lysine/ arginine, aspartic acid/asparagine, leucine/isoleucine, leucine/ valine, alanine/glutamic acid, aspartic acid/glycine, and the like.

In addition, the peptide (b) has immunity induction activity. In the present specification, "immunity induction activity" means an ability to promote the production of antibodies that are specifically bonded to GPNMB. Specifically, as shown in Examples described later, it means that the antibody titer in a case where the peptide is administered is significantly higher than that in a case where the control peptide is administered. The antibody titer can be measured by using a method described in Examples described later.

The peptide contained in the immunity inducer of the present embodiment is expected to be a sequence that is difficult to be presented on a major histocompatibility complex (MHC) from the algorithm analysis. Therefore, an antibody that is specifically bonded to the peptide is usually not present in a living body. However, by administering the immunity inducer of the present embodiment to a subject animal, an antibody that is specifically bonded to the peptide can be produced in the living body of the subject animal with high efficiency.

In addition, the mechanism of immunity induction and removal of aging cells in the living body of the administration target by the immunity inducer of the present embodiment is presumed to be as follows. The administration of the immunity inducer of the present embodiment promotes the production of an antibody that is specifically bonded to GPNMB, the produced anti-GPNMB antibody is bonded to cells expressing GPNMB and acts as a neutralizing antibody, and thus the growth of cells expressing GPNMB is suppressed (neutralizing action). Alternatively, as shown in Examples described later, the produced anti-GPNMB antibody is bonded to cells expressing GPNMB to induce macrophages or natural killer cells (NK cells), and the cells to which the antibody is bonded are killed (Antibody-Dependent-Cellular-Cytotoxicity (ADCC) activity). Alternatively, the produced anti-GPNMB antibody is bonded to cells expressing GPNMB to activate the complement system, causing a series of reactions on the surface of the cell to destroy the cell (Complement-Dependent Cytotoxicity: CDC) activity). This mechanism is only speculated, and even if a desired effect is obtained by a mechanism different from this mechanism, the effect is included in the technical scope.

The peptide (a) or (b) may include L-amino acid, D-amino acid, or a combination thereof. L-amino acid is a naturally present amino acid, and D-amino acid is one in which the chirality of an L-amino acid residue is inverted. In addition, the peptide may also be chemically modified to amplify immunity induction activity or to optimize other physical properties.

In addition, the immunity inducer of the present embodiment may contain an expression vector having a structural gene including the region encoding the peptide as an active ingredient. Such an expression vector having a structural gene encoding the peptide in a living body is also referred to as a "gene vaccine". The immunity inducer of the present embodiment may be a peptide vaccine, or may be a gene vaccine, but a peptide vaccine is preferable.

The Genbank accession number of the human Gpnmb gene is NM_001005340. The Genbank accession number of the mouse Gpnmb gene is NM_053110. SEQ ID NO: 9 shows the base sequence of human Gpnmb cDNA. SEQ ID NO: 10 shows the base sequence of the mouse Gpnmb cDNA. By using a vector including a base sequence of a region encoding amino acid residues at positions 63 to 71 or positions 150 to 159 in the amino acid sequence of the human GPNMB protein, among the base sequence represented by SEQ ID NO: 9, or a base sequence of a region encoding amino acid residues at positions 63 to 71 or positions 150 to 159 in the amino acid sequence of the mouse GPNMB protein, among the base sequence represented by SEQ ID NO: 10, the peptide can be expressed in a living body.

Specifically, examples of the structural gene including a region encoding the peptide include a structural gene including a region including base sequences represented by any of SEQ ID NOS: 11 to 14, a structural gene including a region encoding a peptide having 80% or more, for example, 85% or more, for example, 90% or more, for example, 95% or more identity with base sequences represented by any of SEQ ID NOS: 11 to 14 and having immunity induction activity, or the like. The base sequence represented by SEQ ID NO: 11 is the base sequence of the region encoding the peptide including the amino acid sequence represented by SEQ ID NO: 3. The base sequence represented by SEQ ID NO: 12 is the base sequence of the region encoding the peptide including the amino acid sequence represented by SEQ ID NO: 4. The base sequence represented by SEQ ID NO: 13 is the base sequence of the region encoding the peptide including the amino acid sequence represented by SEQ ID NO: 5. The base sequence represented by SEQ ID NO: 14 is the base sequence of the region encoding the peptide including the amino acid sequence represented by SEQ ID NO: 6.

Here, the sequence identity of the control base sequence with respect to the reference base sequence can be obtained as follows, for example. First, the reference base sequence and the target base sequence are aligned. Here, each base sequence may include a gap so as to maximize the sequence identity. Subsequently, the number of matched bases in the reference base sequence and the target base sequence can be calculated, and the sequence identity can be obtained according to the following formula.

$$\text{“Sequence identity (\%)”} = [\text{number of matched bases}]/[\text{total number of bases in the target base sequence}] \times 100$$

The structural gene including the region encoding the peptide may have other configurations added upstream or downstream of the region encoding the peptide as long as the configurations do not inhibit expression and immunity induction activity of the peptide.

A vector used for the immunity inducer of the present embodiment is not particularly limited as long as the vector is a vector that can be expressed in animal cells (preferably in mammalian cells) to be administered, may be a plasmid vector, may be a virus vector, and a known vector in the field of gene vaccines can be used. Examples of a method using a virus vector include a method of incorporating and introducing a structural gene including a region encoding the peptide into an RNA virus such as a retrovirus, an adenovirus, an adeno-related virus, a herpes virus, a vaccinia virus, a pox virus, a polio virus, a Sindbis virus, or a DNA virus. Among these, a method using a retrovirus, an adenovirus, an adeno-related virus, or a vaccinia virus is particularly preferable. Commercially available products can be preferably used as these vectors. For example, a gene vaccine can be obtained by inserting a structural gene including a region encoding the peptide into a multicloning site of a commercially available vector. The structural gene including the region encoding the peptide can be easily prepared by using a known genetic engineering technique. In addition, incorporation of the structural gene into the vector can be performed using a method well known to those skilled in the art.

In addition, the vector used for the immunity inducer of the present embodiment may include a promoter, a multicloning site, an enhancer, a splicing signal, a poly A addition signal, a selectable marker, and a duplication origin, for example, within a range not inhibiting the expression and immunity induction activity of the peptide.

<Other ingredients>

The peptide contained in the immunity inducer of the present embodiment is preferably used in the form of a peptide-carrier protein complex in which a carrier protein is physically or chemically bonded directly or via a linker or the like in order to enhance immunogenicity. Specific examples of a bonding method include coordination bonding, covalent bonding, hydrogen bonding, hydrophobic interaction, physical adsorption, and the like, and any known bond, linker, and bonding method can be adopted. In addition, the bonding position between the peptide and the carrier protein can be appropriately selected depending on the necessity. In addition, even in a case where there is no physical or chemical bond, a state in which one is restricted from movement of the other due to the three-dimensional structure and can move together is also included in the bonded state in the present embodiment.

A carrier protein is generally a substance that imparts immunogenicity by being bonded to a molecule (hapten) that does not have immunogenicity due to its small molecular weight. Carrier proteins include, but are not limited to, bovine serum albumin (BSA), rabbit serum albumin (RSA), ovalbumin (OVA), keyhole limpet hemocyanin (KLH), thyroglobulin (TG), immunoglobulin, and the like. Among these, keyhole limpet hemocyanin (KLH) is preferable.

In addition, the immunity inducer of the present embodiment can be used as a pharmaceutical composition in combination with an adjuvant. That is, in an embodiment, the present invention provides a pharmaceutical composition for preventing or treating aging-related diseases, which includes the immunity inducer and an adjuvant. With this, the immunity induction activity of the immunity inducer can be enhanced, and the removal of aging cells can be promoted. Therefore, for example, in a case of being used as a pharmaceutical composition for preventing or treating aging-related diseases, the preventive or therapeutic effect of the aging-related diseases can be further enhanced.

As the adjuvant, those used in ordinary vaccine preparations can be used. Specific examples of the adjuvant include alum, CpG oligodeoxynucleotide, dsRNA, montanide, Cervarix, incomplete Freund's adjuvant (Sigma-Aldrich, F5506), complete Freund's adjuvant (Sigma-Aldrich, F5881), TiterMax Gold (TiterMax), and the like. Incomplete Freund's adjuvant (IFA) includes paraffin oil and a surfactant, and complete Freund's adjuvant (CFA) is IFA to which heat-killed bacterium Mycobacterium butyricum is added. In addition, TiterMax Gold is an adjuvant including a water-in-oil emulsion of a nonionic copolymer having a hydrophilic region made of polyoxyethylene and a hydrophobic region made of polyoxypropylene.

Among these, CpG oligodeoxynucleotide is preferable since CpG oligodeoxynucleotide can induce Th1-type helper T cells.

In addition, the immunity inducer of the present embodiment can be used as a pharmaceutical composition in combination with a pharmaceutically acceptable carrier, and is preferably used as a pharmaceutical composition in combination with the adjuvant and a pharmaceutically acceptable carrier. That is, in an embodiment, the present invention provides a pharmaceutical composition for preventing or treating aging-related diseases, which includes the immunity inducer, an adjuvant, and a pharmaceutically acceptable carrier.

The pharmaceutical composition is preferably in a dosage form used parenterally, for example, an injection, an external skin preparation, and the like. Specific examples of the external skin preparation include dosage forms such as ointments and patches.

As the pharmaceutically acceptable carrier, those usually used for the preparation of a pharmaceutical composition can be used without particular limitation. Specific examples thereof include a binder such as gelatin, cornstarch, gum Tragacanth, and gum Arabic; an excipient such as starch and crystalline cellulose; a swelling agent such as alginic acid; a solvent for injection such as water, ethanol, and glycerin; an adhesive such as rubber-based adhesive and silicone-based adhesive, and the like.

The pharmaceutical composition may contain an additive. Examples of the additive include a lubricant such as calcium stearate and magnesium stearate; a sweetener such as sucrose, lactose, saccharin, and maltitol; a flavoring agent such as peppermint and red mono oil; a stabilizer such as benzyl alcohol and phenol; a buffering agent such as phosphate and sodium acetate; a solubilizing agent such as benzyl benzoate and benzyl alcohol; an antioxidant; a preservative; and the like.

The pharmaceutical composition may be used in combination with at least one selected from the group consisting of therapeutic agents having an aging cell-removing effect other than the immunity inducer (hereinafter, may be abbreviated as "aging cell-removing agents") and therapeutic agents for other diseases. The immunity inducer and other agents may be the same preparations, or may be different preparations. In addition, each preparation may be administered by the same administration pathway, or may be administered by a different administration pathway. In addition, each preparation may be administered simultaneously, may be administered sequentially, or may be administered separately after a certain time or period. In an embodiment, the immunity inducer and other agents may be in the form of a kit containing these.

<Administration method>

Subjects to be administered include, but are not limited to, humans, monkeys, dogs, cows, horses, sheep, pigs, rabbits, mice, rats, guinea pigs, hamsters, and cells thereof. Among these, mammals or mammalian cells are preferable, and humans or human cells are particularly preferable.

The administration pathway is preferably a parenteral administration pathway such as subcutaneous administration, transdermal administration, intramuscular administration, intraperitoneal administration, intravenous administration, and intraarterial administration.

The dose of the immunity inducer varies depending on the symptoms, age, gender, and the like of the administration subject and cannot be unconditionally determined, but is not particularly limited as long as the dose can induce an immune response in the living body of the administration subject. For example, the mass of the peptide (preferably peptide-carrier protein complex) per dose is 0.1 μg or more and 100 mg or less, and is preferably 1 μg or more and 10 mg or less. The administration of the immunity inducer may be a single dose, or may be a plurality of doses. In the case of a plurality of doses, for example, the immunity inducer can be administered twice or three times at intervals of two weeks or one month, and then once every six months or one year.

«Other Embodiments»

In an embodiment, the present invention provides a method of preventing or treating a patient with an aging-related disease, including administering an effective amount of any of the peptide (a) or (b), or an expression vector having a structural gene including a region encoding the peptide to a patient in need of treatment. As the aging-related diseases, the same ones as described above can be used.

In an embodiment, the present invention provides a method of preventing or treating aging-related diseases, including administering an effective amount of a peptide-carrier protein complex of any of the peptide (a) or (b) and a carrier protein bonded to the peptide to a patient in need of treatment. As the carrier protein, the same proteins as described above can be used.

In an embodiment, the present invention provides a method of preventing or treating aging-related diseases, including administering an effective amount of a pharmaceutical composition containing any of the peptide (a) or (b), or an expression vector having a structural gene including a region encoding the peptide, and an adjuvant to a patient in need of treatment. As the adjuvant, the same adjuvants as those described above can be used.

In an embodiment, the present invention provides a method of preventing or treating aging-related diseases, including administering an effective amount of a pharmaceutical composition containing a peptide-carrier protein complex of any of the peptide (a) or (b) and a carrier protein bonded to the peptide and an adjuvant to a patient in need of treatment.

In an embodiment, the present invention provides any of the peptide (a) or (b) for preventing or treating aging-related diseases, or an expression vector having a structural gene including a region encoding the peptide.

In an embodiment, the present invention provides a peptide-carrier protein complex including any of the peptide (a) or (b) for preventing or treating aging-related diseases and a carrier protein bonded to the peptide.

In an embodiment, the present invention provides a pharmaceutical composition for preventing or treating aging-related diseases, the pharmaceutical composition containing any of the peptide (a) or (b), or an expression vector having a structural gene including a region encoding the peptide, and an adjuvant.

In an embodiment, the present invention provides a pharmaceutical composition for preventing or treating aging-related diseases, the pharmaceutical composition containing a peptide-carrier protein complex including any of the peptide (a) or (b) and a carrier protein bonded to the peptide and an adjuvant.

In an embodiment, the present invention provides a use of any of the peptide (a) or (b) for producing a prophylactic or therapeutic agent for aging-related diseases, or an expression vector having a structural gene including a region encoding the peptide.

In an embodiment, the present invention provides a use of a peptide-carrier protein complex including any of the peptide (a) or (b) for producing a prophylactic or therapeutic agent for aging-related diseases and a carrier protein bonded to the peptide.

In an embodiment, the present invention provides a use of any of the peptide (a) or (b) for producing a prophylactic or therapeutic agent for aging-related diseases, or an expression vector having a structural gene including a region encoding the peptide, and an adjuvant.

EXAMPLES

Hereinafter, the present invention will be described based on examples, but the present invention is not limited to the following examples.

(Animal model)

All animal tests were reviewed by the Niigata University Animal Health Research Institute Utilization Review Board and approved by the President of Niigata University. C57BL/6 mice were purchased from SLC Japan (Shizuoka, Japan). Unless otherwise specified, the mice used in the following tests were maintained on a high-fat diet (HFD, CLEA Japan) or a normal diet (NC) until 4 to 12 weeks of age, and maintained up to 4 to 16 weeks of age for peptide inoculation.

In addition, ApoE-deficient mice (ApoE KO mice) (ApoE-/-, C57BL/6 background) used in the following tests were obtained from Jackson Laboratory. In addition, ApoE KO mice exhibit spontaneous arteriosclerotic lesions even in normal dietary captivity.

ApoE/Gpnmb-DTR mice (C57BL/6 background) were prepared by mating ApoE KO mice with Gpnmb-DTR mice described later. ApoE/Gpnmb-DTR mice were given HFD from 4 weeks of age, treated with DT at 12 and 14 weeks of age, and analyzed at 16 weeks of age.

In addition, a gene-modified mouse model in which the DTR (human HB-EGF I117V/L148V) and luciferase used in the following tests were expressed at the $p19^{Arf}$ locus (hereinafter, abbreviated as "$p19^{Arf}$-DTR-Luc mouse") (C57BL/6 background) was prepared using a method described in Reference 1 (Hashimoto M et al., "Elimination of p19ARF-expressing cells enhances pulmonary function in mice.", JCI Insight, Vol. 1, Issu 12: e87732, 2016.).

In addition, Zmpste24-deficient (KO) mice (model of Hutchinson-Gilford progeria syndrome, MGI: 2158363) were used as a model of premature aging mice. Generation and genotyping of Zmpste24 KO mice followed the previous report (http://www.informatics.jax.org/allele/MGI:2158363).

(Statistical analysis)

In addition, statistical analysis of the test data shown below was performed using SPSS software (version 24 or 25). All data are obtained from different biological iterations, and are shown as boxes and whisker plots indicating the scope of data (whiskers), 25th and 75th percentiles (boxes), and median value (solid line) or mean value ± standard errors. Deviation values and abnormal values were excluded by box plot analysis. Differences between groups were tested using a two-way student's t-test or two-way analysis of variance (two-way ANOVA), subsequently, comparison between two or more groups was performed using Tukey's multiple comparison test, or analysis of repeated measures for comparison over time was performed using Tukey's multiple comparison test. The survival curve was drawn by the Kaplan-Meier method and compared with a log-rank test. In all analyses, $P < 0.05$ was considered to be statistically significant.

[Reference Example 1]

1. Establishment of genetically modified mice capable of removing GPNMB protein-positive cells

The inventors considered that the GPNMB protein could be a target molecule for aging cell death induction therapy (senolytic therapy), and a genetically modified mouse model in which DTR (human HB-EGF I117V/L148V) and luciferase were expressed at the Gpnmb locus (hereinafter, abbreviated as "Gpnmb-DTR mouse" or "Gpnmb-DTR-Luc mice") was established.

First, BAC clone (RP23-284O3, ~ 200 kb) including a complete 21.7 kb mouse Gpnmb genome sequence having an additional 97 kb 5'-flanking region and 80 kb 3'-flanking region was purchased from Advanced Geno Techs.

A chimeric Gpnmb-DTR-2A-luciferase BAC transgenic construct (hereinafter, may be abbreviated as "BAC transgenic construct") having the configuration shown below was prepared by recombination (refer to Fig. 1A).

Construct configuration: Tandem cassette-2A peptide gene-luciferase reporter gene-Gpnmb gene of human heparin-binding epidermal cell growth factor-like growth factor (human HB-EGF) having DTR-generating I117V / L148V mutation

Specifically, the DTR-2A-luciferase gene was prepared by transferring to a Gpnmb BAC clone using a Red/ET counter-selection BAC modification kit (Gene Bridges). A Gpnmb-DTR mouse was prepared by pronuclear injection of the obtained BAC transgenic construct into C57BL/6 mouse embryos. Genetic modification by the BAC transgenic construct was evaluated by Southern blotting of tail DNA digested with Hinc I and probing of a $^{32}P$-labeled luciferase gene fragment. Twenty strains of Gpnmb-DTR mouse were obtained and two strains with high copy counts (~ 10 copies, 2-2 strains and 5-4 strains) were selected for analysis. Two independent mouse strains were confirmed to exhibit similar phenotypes. Unless otherwise stated, Gpnmb-DTR mouse was maintained on HFD until 4 to 16 weeks of age to generate a dietary obesity model mouse.

2. Analysis of Gpnmb-dependent luciferase activity

Gpnmb-DTR mouse bred by feeding normal diet or HFD for 4 weeks, and Gpnmb-DTR mouse bred by feeding HFD for 4 weeks, and then injecting DT (50 μg/kg body weight) at 2-week intervals (12 weeks of age and 14 weeks of age) intraperitoneally twice while continuing to feed HFD (16 weeks of age) were subjected to in vivo luciferase imaging analysis using the IVIS imaging system (Perkin Elmer). Mice were shaved ventrally, anesthetized with isoflurane (Wako Pure Chemicals Industries), and injected intraperitoneally with luciferin (150 mg/kg body weight) according to the manufacturer’s instructions (Vivo-Glo; Promega). Luciferase activity was monitored 5 minutes after luciferin administration. Signals were analyzed using Living Image software (Perkin Elmer) to quantify luciferase activity. The results are shown in Fig. 1B. In Fig. 1B, "Before" is a Gpnmb-DTR mouse before HFD was fed (6 weeks of age), "HFD" is a Gpnmb-DTR mouse that was fed with HFD from 6 weeks of age and bred for 6 weeks (12 weeks of age), and "After DT" is a Gpnmb-DTR mouse that was bred by feeding HFD for 4 weeks and then injecting DT (50 μg/kg body weight) at 2-week intervals (12 weeks of age and 14 weeks of age) intraperitoneally twice while continuing to feed HFD (16 weeks of age).

From Fig. 1B, when HFD was administered to Gpnmb-DTR mice, a strong abdominal luciferase signal was detected, and administration of DT attenuated abdominal luciferase activity. That is, in the obtained Gpnmb-DTR mouse, cells expressing the GPNMB protein (hereinafter, may be referred to as "GPNMB-positive cells") could be detected by monitoring the luciferase activity, and GPNMB-positive cells could be removed by administration of diphtheria toxin (DT).

3. SA-β-gal staining

Aging-related acidic β-galactosidase (SA-β-gal) activity in adipose tissue was tested. Briefly, adipose tissue newly isolated from each Gpnmb-DTR mouse was incubated at 37°C for 2 hours in a β-gal staining solution (containing 1 mg/ml of 5-bromo-4-chloro-3-indaryl β-D-galactopyranoside (X-gal), 5 mmol/L potassium ferrocyanide, 150 mmol/L of sodium chloride, 2 mmol/L of magnesium chloride, 0.01% of sodium deoxycholate, and 0.02% of nonidet-40). Then, the stained adipose tissue was captured (refer to Fig. 1C). SA-β-gal stainability was quantified by Image-J software (refer to Fig. ID). In addition, in Figs. 1C and 1D, "DTR+" is a Gpnmb-DTR mouse that was bred by feeding HFD from 4 weeks of age and then injecting DT (50 μg/kg body weight) at 2-week intervals (12 weeks of age and 14 weeks of age) intraperitoneally twice while continuing to feed HFD (16 weeks of age), and "DTR-" is a littermate mouse of Gpnmb-DTR mouse that was bred by feeding HFD from 4 weeks of age and then injecting DT (50 μg/kg body weight) at 2-week intervals (12 weeks of age and 14 weeks of age) intraperitoneally twice while continuing to feed HFD (16 weeks of age, control/wild type). For "DTR+" and "DTR-", the same mouse groups are shown below unless otherwise specified. In Fig. 1C, the scale bar is 5 mm.

4. RNA analysis

Total RNA (1 μg) was isolated from adipose tissue samples using RNA-Bee (TEL-TEST Inc.). Real-time PCR (qPCR) was performed using a Light Cycler 480 (Roche) equipped with a universal probe library and a Light Cycler 480 probe master (Roche) according to the manufacturer’s instructions. The primers and the sequences thereof are shown in Table 1 below. Rplp0 was used as an internal control. The results are shown in Fig. 1E.

[Table 1]

| Gene name | Type | Base sequence | SEQ ID NO |
|---|---|---|---|
| Gpnmb | Forward primer | 5’ -ACGGCAGGTGGAAGGACT -3’ | 15 |
| | Reverse primer | 5’ -CGGTGAGTCACTGGTCAGG-3’ | 16 |

(continued)

| Gene name | Type | Base sequence | SEQ ID NO |
|---|---|---|---|
| Cdkn1a | Forward primer | 5'-TCCACAGCGATATCCAGACA-3' | 17 |
| | Reverse primer | 5'-GGACA TCACCAGGA TTGGAC-3' | 18 |
| Cdkn2a | Forward primer | 5'-GGGTTTTCTTGGTGAAGTTCG-3' | 19 |
| | Reverse primer | 5'-TTGCCCATCATCATCACCT-3' | 20 |
| Rplp0 | Forward primer | 5'-GATGCCCAGGGAAGACAG-3' | 21 |
| | Reverse primer | 5'-ACAATGAAGCATTTTGGATAA-3' | 22 |

5. Metabolic test

Mice were bred individually from 1 week before the test. On the day of the glucose tolerance test (GTT), mice were fasted for 6 hours, followed by intraperitoneal injection of glucose at a dose of 1 g/kg (body weight) in the early afternoon. For the insulin tolerance test (ITT), mice were intraperitoneally administered with human insulin (1 U/kg (body weight)). After administration of glucose or insulin, tail venous blood was collected 0, 15, 30, 60 and 120 minutes later, and the blood glucose level was measured with a glucose analyzer (Sanwa Chemical Research Institute). The results are shown in Fig. IF.

From Figs. 1C to 1E, in DTR+ Gpnmb-DTR mice (16 weeks of age), decreased SA-β-gal activity and downregulation of Gpnmb, Cdknla, and Cdkn2a expression were shown, and aging phenotype of visceral adipose tissue was improved.

In addition, from Fig. 1F, it was shown that removal of GPNMB-positive cells resulted in a significant improvement in metabolic abnormality induced by ingestion of HFD.

6. Detection of arteriosclerotic plaque

Arteriosclerotic plaques were detected by Oil Red O staining. Briefly, autopsy removed adventitial fat to open the entire aorta and fixed the aorta flat in 4 v/v% paraformaldehyde at room temperature for 12 hours. Subsequently, the fixed aorta was washed by immersing in 60 v/v% isopropyl alcohol for 1 minute. Subsequently, the aorta was incubated at 37°C for 15 minutes in 0.5% of Oil Red O (Sigma-Aldrich) solution (diluted with 60 v/v% isopropyl alcohol). After staining, the sample was briefly immersed in a 60 v/v% isopropyl alcohol solution and then washed with double distilled water. Atherosclerotic lesions were stained red with Oil Red O (refer to Fig. 1G). For the Oil Red O stained region, the plaque region was quantified by Image-J software (refer to Fig. 1H). In Figs. 1G and 1H, "DTR+" is an ApoE/Gpnmb-DTR mouse that was bred by feeding HFD from 4 weeks of age and then injecting DT (50 μg/kg body weight) at 2-week intervals (12 weeks of age and 14 weeks of age) intraperitoneally twice while continuing to feed HFD (16 weeks of age), and "DTR-" is a littermate mouse of ApoE/Gpnmb-DTR mouse that was bred by feeding HFD from 4 weeks of age and then injecting DT (50 μg/kg body weight) at 2-week intervals (12 weeks of age and 14 weeks of age) intraperitoneally twice while continuing to feed HFD (16 weeks of age, control/wild type).

From Figs. 1G and 1H, in the DTR+ ApoE/Gpnmb-DTR mouse, removal of GPNMB-positive cells significantly reduced arteriosclerotic plaques. From this, it was suggested that the GPNMB protein may be a useful target for aging cell death induction therapy.

[Example 1]

1. Construction of peptide vaccine

Subsequently, a peptide vaccine targeting the GPNMB protein was developed. Specifically, for the extracellular domain (positions 1 to 487 from N-terminal) of human GPNMB protein, algorithm analysis was performed using information of MHC presentation sequence (T cell epitope) and B cell epitope acquired from IEDB (http://www.iedb.org/home_v3.php, search date: May 15, 2017), and as the top two peptides, peptides including a region at positions 63 to 71 in the amino acid sequence of the human GPNMB protein (SEQ ID NO: 3: KRGDMRWKN) or an amino acid sequence at positions 150 to 159 (SEQ ID NO: 5: QSHHNVFPDG) were selected. In addition, by homology analysis of human GPNMB protein and mouse GPNMB protein, the peptide including the amino acid sequence represented by SEQ ID NO: 4 (RRGDGRWKD) or SEQ ID NO: 6 (RSQHLRFPDR) corresponding to the two regions in mouse GPNMB protein was used in the following test. In the following test, the peptide including the amino acid sequence represented by SEQ ID NO: 4 (RRGDGRWKD) is referred to as peptide vaccine 1, and the peptide including the amino acid sequence represented by SEQ ID NO: 6 (RSQHLRFPDR) is referred to as peptide vaccine 2.

First, the antibody titers of the obtained two peptide vaccines were examined. Peptide vaccine 1 or peptide vaccine 2 (20 μg) and Titer Max Gold adjuvant (Titer Max USA) conjugated with KLH as carrier protein for induction of cytotoxic antibody were mixed in two syringes according to the manufacturer's protocol for emulsification. Subsequently, eight-week-old C57BL/6 mice fed with HFD from 4 weeks of age were subcutaneously injected with KLH-binding antigen peptide (20 μg) with a Titer Max Gold adjuvant to induce cytotoxic antibodies. C57BL/6 mice in the control group were subcutaneously injected with a KLH solution (hereinafter, referred to as "control vaccine") mixed with Titer Max Gold in an equal amount. Plasma was collected from the tail vein of each mouse immediately after administration of the peptide vaccine and after 2, 4, and 6 weeks for antibody titer analysis. The antibody titer produced by injection of each peptide was measured by ELISA. Specifically, first, a 96-well microtiter plate (Nunc-Immuno MicroWell 96-well solid plate, Thermo Scientific) was coated using a carbonate buffer containing peptide vaccine 1 or peptide vaccine 2 bonded to bovine serum albumin (BSA). After coating, plasma collected from immunized mice was added to the wells and incubated overnight. Subsequently, the plate was incubated with a secondary antibody (ECL anti-mouse IgG, horseradish peroxidase-binding all sheep antibody, GE Healthcare). After reacting with the TMB substrate (Sigma-Aldrich, T0440), the optical density was measured at 450 nm (OD450) using a microtiter plate reader (iMark microplate reader, Bio-Rad). The results are shown in Fig. 2A.

From Fig. 2A, the antibody titer against GPNMB protein by peptide vaccine 1 was significantly higher and the antibody titer against GPNMB protein by peptide vaccine 2 was slightly higher than that of the control group to which the control vaccine was administered.

2. Analysis of Gpnmb-dependent luciferase activity

Luciferase activity was observed using the same method as in "2." of Reference Example 1, except that Gpnmb-DTR mouse (16 weeks of age) bred by feeding HFD from 4 weeks of age and administered with peptide vaccine 1 or a control vaccine by subcutaneous injection at a time point of 8 weeks of age was used. The results are shown in Fig. 2B. In Fig. 2B, "Cont vac" is a Gpnmb-DTR mouse administered with a control vaccine, and "Gpnmb vac" is a Gpnmb-DTR mouse administered with peptide vaccine 1.

3. RNA analysis

RNA analysis of Gpnmb, Cdknla, and Cdkn2a was performed using the same method as in "4." of Reference Example 1, except that adipose tissue collected from C57BL/6 mice (16 weeks of age) bred by feeding HFD from 4 weeks of age and administered with peptide vaccine 1 or a control vaccine by subcutaneous injection at a time point of 8 weeks of age was used. The results are shown in Fig. 2C. In Fig. 2C, "Cont vac" is a C57BL/6 mouse administered with a control vaccine, and "Gpnmb vac" is a C57BL/6 mouse administered with peptide vaccine 1.

4. Western blotting analysis

For adipose tissue collected from C57BL/6 mice (16 weeks of age) bred by feeding HFD from 4 weeks of age and administered with peptide vaccine 1 or a control vaccine by subcutaneous injection at a time point of 8 weeks of age, whole-cell lysates were prepared in lysis buffer (containing 10 mmol/L of Tris-HCl (pH 8), 140 mmol/L of sodium chloride, 5 mmol/L of EDTA, 0.025% of $NaN_3$, 1 w/v% of Triton X-100, 1 w/ v% of deoxycholic acid, 0.1 w/v% of SDS, 1 mmol/L of phenylmethylsulfonyl fluoride (PMSF), 5 μg/mL of leypeptin, 2 μg/mL of aprotinin, 50 mmol/L of NaF, and 1 mmol/L of $Na_2VO_3$). Then the lysates (40 to 50 μg) was separated by SDS-PAGE, the protein was transferred to a PVDF membrane (Millipore), and this was incubated with a primary antibody, followed by horseradish peroxidase (Jackson)-binding anti-rabbit immunoglobulin G (Jackson ImmunoResearch). GPNMB and β-actin were detected by enhanced chemiluminescence (Amersham). As a primary antibody, an anti-GPNMB antibody (AF2330, R & D Systems) (1/1000 dilution) was used, and as a control, anti-β-actin antibody (# 4967, Cell Signaling) (1/5000 dilution) was used. As a secondary antibody, peroxidase-binding AffiniPure goat anti-rabbit IgG (H + L) (111-035-003, Jackson ImmunoResearch) (1/10000 dilution) was used. The results are shown in Fig. 2D. In Fig. 2D, "Cont vac" is a C57BL/6 mouse administered with control vaccine, and "Gpnmb vac" is a C57BL/6 mouse administered with peptide vaccine 1.

From Figs. 2B to 2D, for both Gpnmb-DTR mouse and C57BL/6 mouse bred by feeding HFD and administered with peptide vaccine 1, it was suggested that both an expression amount of GPNMB protein and luciferase activity related to the expression amount decreased, and GPNMB-positive cells were removed by the administration of peptide vaccine 1.

5. ADCC (Antibody-Dependent-Cellular-Cytotoxicity) activity analysis

ADCC (Antibody-Dependent-Cellular-Cytotoxicity) was evaluated using a CytoTox 96 non-radioactive cytotoxicity assay kit (Promega). Specifically, natural killer (NK) cells were isolated from C57BL/6 mice as effector cells using Ficoll-Paque Plus (GE Healthcare) and EasySep mouse NK cell isolation kit (STEMCELL). Subsequently, as target cells, replicative aging adipose precursor cells derived from C57BL/6 mice were seeded on a 96-well plate so as to have $10^4$ cells per well. Subsequently, purified IgG antibody obtained from C57BL/6 mice bred by feeding HFD from 4 weeks of age and administered with peptide vaccine 1 or a control vaccine by subcutaneous injection at a time point of 8 weeks of age was added to each well, and incubated at 37°C for 30 minutes. After incubation, NK cells were seeded in each well so that a ratio of the cell number of effector cells to the target cells (effector cells/target cells) was 1/1. Subsequently, the plates were incubated at 37°C for an additional 4 hours. Subsequently, lactate dehydrogenase released in a supernatant was detected using a microtiter plate reader (iMark microplate reader, Bio-Rad). To evaluate cell death, a ratio of lysed cells to the total number of cultured cells was calculated. The results are shown in Fig. 2E. In Fig. 2E, "Cont vac" is a C57BL/6 mouse administered with the control vaccine, and "Gpnmb vac" is a C57BL/6 mouse administered with peptide vaccine 1.

From Fig. 2E, it was confirmed that in the antibody derived from the mouse administered with the peptide vaccine 1, the anti-GPNMB antibody contained in the antibody induces NK cells by being specifically bonded to the aging adipose precursor cells expressing GPNMB, and exhibits antibody-dependent cellular cytotoxicity (ADCC activity) that kills the cells to which the antibody is bonded.

6. Analysis of $p19^{Arf}$-dependent luciferase activity

Luciferase activity was observed using the same method as in "2." of Reference Example 1, except that $p19^{Arf}$-DTR-Luc mouse (21 weeks of age) bred by feeding HFD for 8 weeks and administered with peptide vaccine 1 or a control vaccine by subcutaneous injection at a time point of 13 weeks of age was used. The results are shown in Fig. 2F. In Fig. 2F, "Cont vac" is a $p19^{Arf}$-DTR-Luc mouse administered with the control vaccine, and "Gpnmb vac" is a $p19^{Arf}$-DTR-Luc mouse administered with peptide vaccine 1.

From Fig. 2F, a decrease in $p19^{Arf}$-dependent luciferase activity in visceral adipose tissue was confirmed by administration of peptide vaccine 1. It is known that induction of the expression of the $p19^{Arf}$ gene ($p14^{Arf}$ gene in humans) encoded by the Cdkn2a locus in mammals inhibits the activity of ubiquitin ligase Mdm2, suppresses the degradation of tumor suppressor protein p53, and causes cellular aging. Therefore, it was confirmed that aging cells were removed by the administration of the peptide vaccine 1.

7. SA-β-gal staining

SA-β-gal staining was performed using the same method as in "3." of Reference Example 1, except that adipose tissue collected from C57BL/6 mice (16 weeks of age) bred by feeding HFD from 4 weeks of age and administered with peptide vaccine 1 or a control vaccine by subcutaneous injection at a time point of 8 weeks of age was used. The results are shown in Fig. 2G. The SA-β-gal stainability was quantified by Image-J software (refer to Fig. 2H). In Figs. 2G and 2H, "Cont vac" is a C57BL/6 mouse administered with the control vaccine, and "Gpnmb vac" is a C57BL/6 mouse administered with the peptide vaccine 1. In addition, in Fig. 2G, the scale bar indicates 5 mm.

From Figs. 2G and 2H, administration of peptide vaccine 1 showed a decrease in SA-β-gal activity. In addition, downregulation of the expression of Cdkn1a and Cdkn2a was shown in Fig. 2C. From these findings, it was clarified that aging phenotype of visceral adipose tissue was improved by the administration of peptide vaccine 1.

8. Metabolic test

A metabolic test was performed using the same method as in "5." of Reference Example 1, except that C57BL/6 mice (16 weeks of age) bred by feeding HFD from 4 weeks of age and administered with peptide vaccine 1, peptide vaccine 2, or control vaccine by subcutaneous injection at a time point of 8 weeks of age were used. The results are shown in Fig. 2I. In Fig. 2I, "Cont vaccine" is a C57BL/6 mouse administered with a control vaccine, "Gpnmb vaccine 1" is a C57BL/6 mouse administered with peptide vaccine 1, and "Gpnmb vaccine 2" is a C57BL/6 mouse administered with peptide vaccine 2.

From Fig. 2I, in a C57BL/6 mouse administered with peptide vaccine 1 or peptide vaccine 2, a metabolic abnormality induced by HFD intake was significantly improved compared to the C57BL/6 mouse administered with the control vaccine.

9. Detection of arteriosclerotic plaque and analysis of Gpnmb-dependent luciferase activity

Detection of arteriosclerotic plaque and analysis of Gpnmb-dependent luciferase activity were performed using the same method as in "6." and "2." of Reference Example 1, except that ApoE/Gpnmb-DTR mice (16 weeks of age) bred while feeding HFD for 12 weeks from 4 to 16 weeks of age and administered with peptide vaccine 1 or a control vaccine by subcutaneous injection at a time point of 8 weeks of age were used (refer to Fig. 2J). The Oil Red O stained region was quantified by Image-J software (refer to Fig. 2K). In Figs. 2J and 2K, "Cont vac" is an ApoE/Gpnmb-DTR mouse administered with a control vaccine, and "Gpnmb vac" is an ApoE/Gpnmb-DTR mouse administered with peptide vaccine 1.

From Figs. 2J and 2K, treatment of ApoE/Gpnmb-DTR mice administered with peptide vaccine 1 significantly decreased the development of atheroma and decreased Gpnmb-dependent luciferase activity in the aorta.

10. Physiological analysis

C57BL/6 mice bred by feeding a normal diet and administered with peptide vaccine 1 or a control vaccine by subcutaneous injection at a time point of 50 weeks of age were used. Open field tests were performed before and after vaccination (50 and 70 weeks of age). The open field test is a test that measures voluntary activity in a novel environment. Each mouse was placed in an open field and tested for 6 minutes. The total action time (seconds) and average speed (cm/sec) of each mouse during the test were measured. The difference between the measurement results at 50 weeks of age and the measurement results at 70 weeks of age is shown in Fig. 2L. In Fig. 2L, "Cont vac" is a C57BL/6 mouse administered with the control vaccine, and "Gpnmb vac" is a C57BL/6 mouse administered with the peptide vaccine 1.

In Fig. 2L, the results in the open field test were significantly improved by administration of peptide vaccine 1.

11. Survival curve

Zmpste24 KO mice were bred on normal diet. For some Zmpste24 KO mice, peptide vaccine 1 was administered by subcutaneous injection at a time point of 10 weeks of age (about 50 weeks of age in C57BL/6 mice). Survival curves were calculated by the Kaplan-Meier method and compared by a log-rank test (refer to Fig. 2M).

From Fig. 2M, it was shown that in a Zmpste24 KO mouse, which is a mouse model of premature aging, the lifespan is extended regardless of gender even in a case where peptide vaccine 1 is administered in middle age (about 50 weeks of age).

From the above results, it was demonstrated that removal of GPNMB-positive cells can improve the development of atheroma and metabolic dysfunction induced by ingestion of HFD. In addition, removal of GPNMB-positive cells using a peptide vaccine was able to attenuate pathological aging in aged mice and prolong the lifespan of prematurely aged mice.

Aging cells are known to be resistant to apoptosis by upregulating anti-apoptotic factors. Therefore, most aging cell death induction agents (senolytic agents) in the related art have been developed based on the ability to inhibit the anti-apoptotic pathway. However, targeting the GPNMB protein could be a new strategy for aging cell death induction therapy (senolytic therapy) for age-related diseases.

It has been reported that the GPNMB protein can be a negative regulator of T lymphocyte activation by being bonded to Syndecan-4 on T cells. Therefore, an immunity inducer using a peptide vaccine is considered to enhance an endogenous aging cell death induction immune system (senolytic immune system), thereby promoting the removal of aging cells, and pathological aging may be improved.

INDUSTRIAL APPLICABILITY

The immunity inducer of the present embodiment is effective for preventing or treating aging-related diseases.

## Claims

**1.** An immunity inducer comprising, as an active ingredient:
any of a peptide (a) or (b) below; or an expression vector having a structural gene including a region encoding the peptide,

(a) a peptide including an amino acid sequence represented by SEQ ID NO: 1 or 2;
(b) a peptide having immunity induction activity and including an amino acid sequence in which one or several amino acids are deleted, substituted, or added in the amino acid sequence represented by SEQ ID NO: 1 or 2.

**2.** The immunity inducer according to Claim 1,
wherein the peptide includes an amino acid sequence represented by any of SEQ ID NOS: 3 to 6.

**3.** The immunity inducer according to Claim 1 or 2, comprising:
the peptide as an active ingredient.

**4.** The immunity inducer according to any one of Claims 1 to 3,
wherein a carrier protein is further bonded to the peptide.

**5.** The immunity inducer according to any one of Claims 1 to 4, which is used for removing aging cells.

**6.** A pharmaceutical composition for preventing or treating aging-related diseases, comprising:

an immunity inducer of any one of Claims 1 to 5; and
an adjuvant.

FIG. 1A

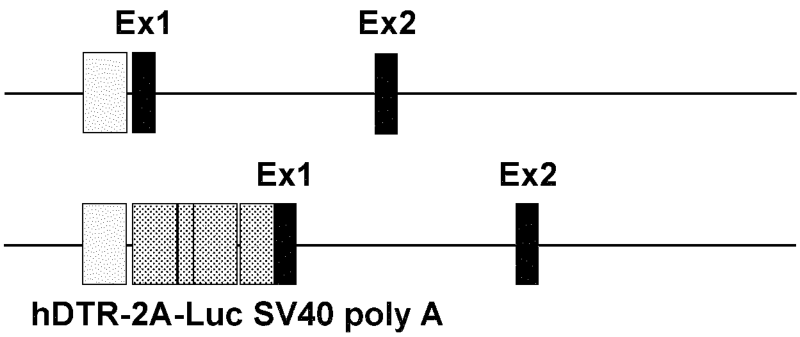
Ex1
Ex2
Ex1
Ex2
hDTR-2A-Luc SV40 poly A

FIG. 1B

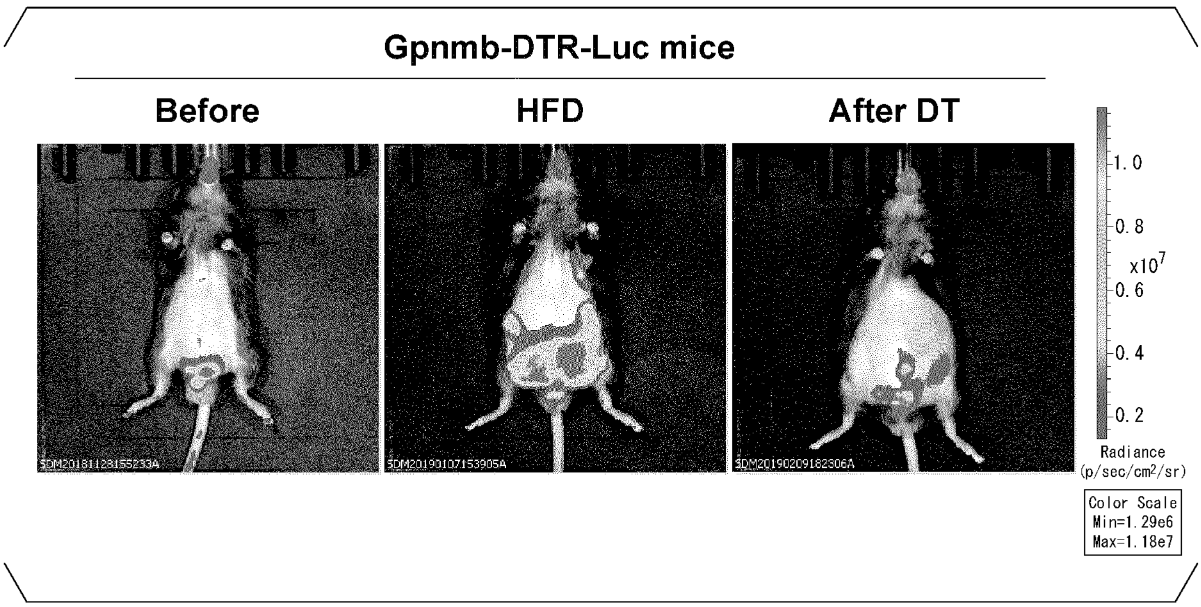
Gpnmb-DTR-Luc mice
Before
HFD
After DT
1.0
0.8
x10^7
0.6
0.4
0.2
Radiance (p/sec/cm²/sr)
Color Scale
Min=1.29e6
Max=1.18e7

FIG. 1C

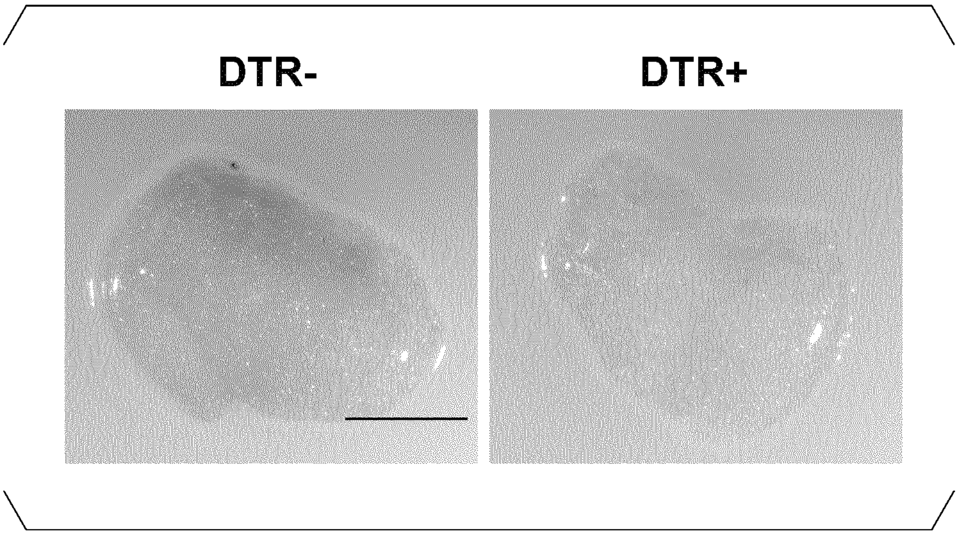


FIG. 1D

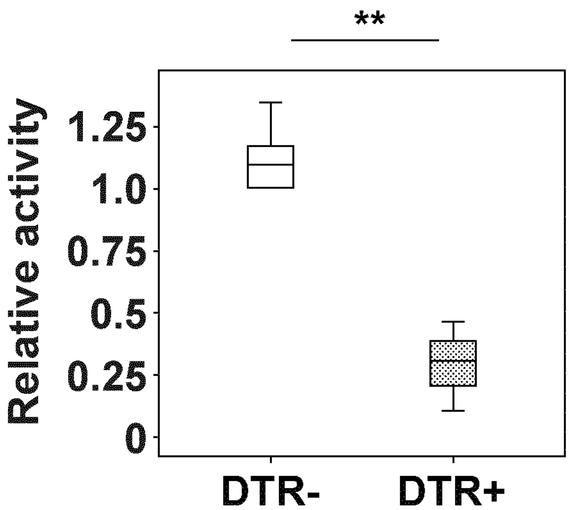

FIG. 1E

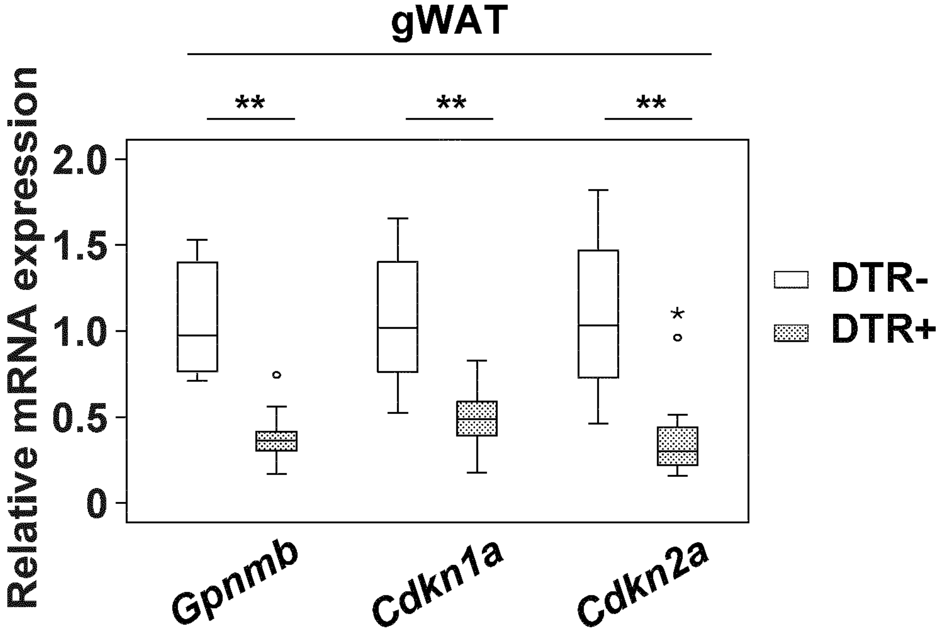
gWAT
**
**
**
Relative mRNA expression
2.0
1.5
1.0
0.5
0
Gpnmb
Cdkn1a
Cdkn2a
DTR-
DTR+

FIG. 1F

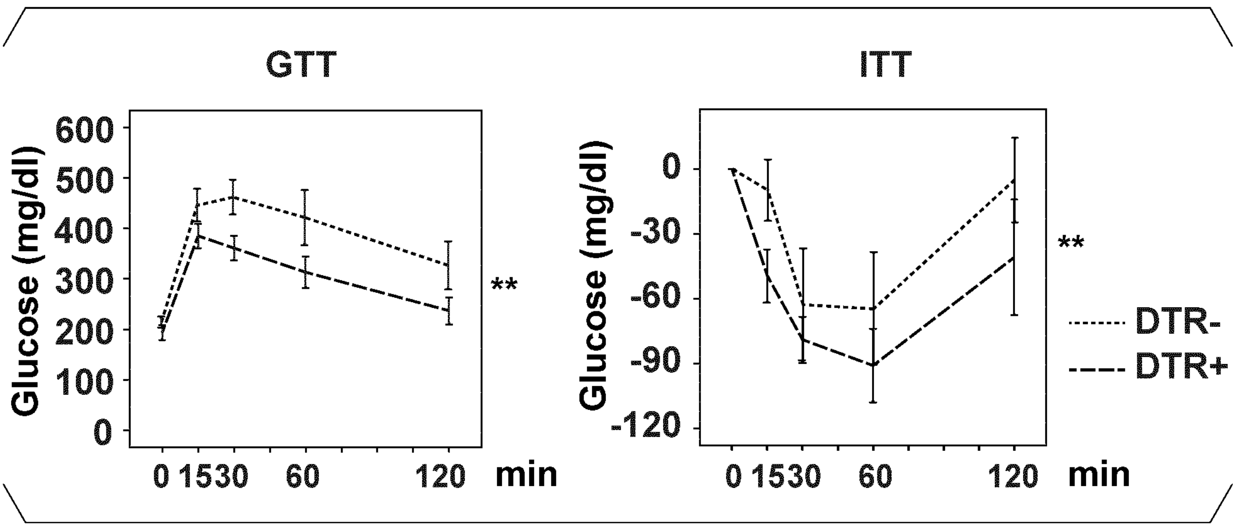
GTT
ITT
Glucose (mg/dl)
600
500
400
300
200
100
0
0 1530 60 120 min
**
0
-30
-60
-90
-120
DTR-
DTR+

FIG. 1G

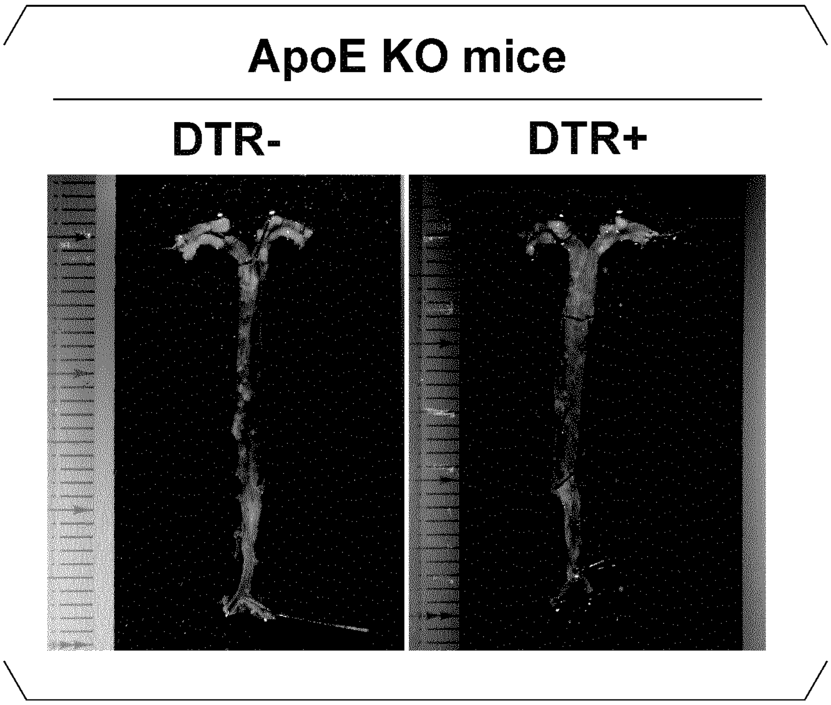
ApoE KO mice
DTR-
DTR+

FIG. 1H

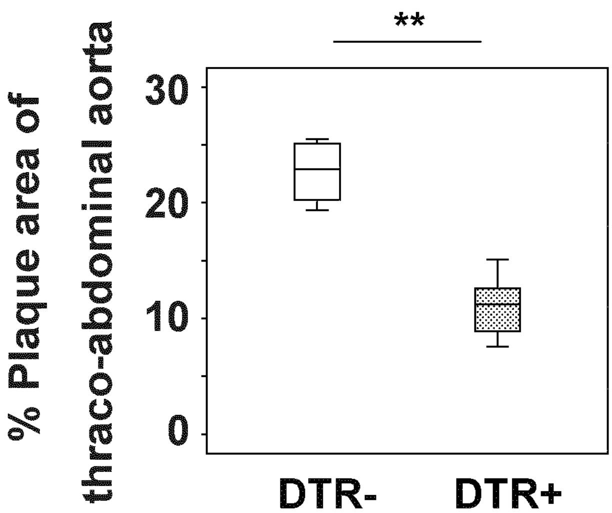
**
% Plaque area of thraco-abdominal aorta
30
20
10
0
DTR-
DTR+

FIG. 2A

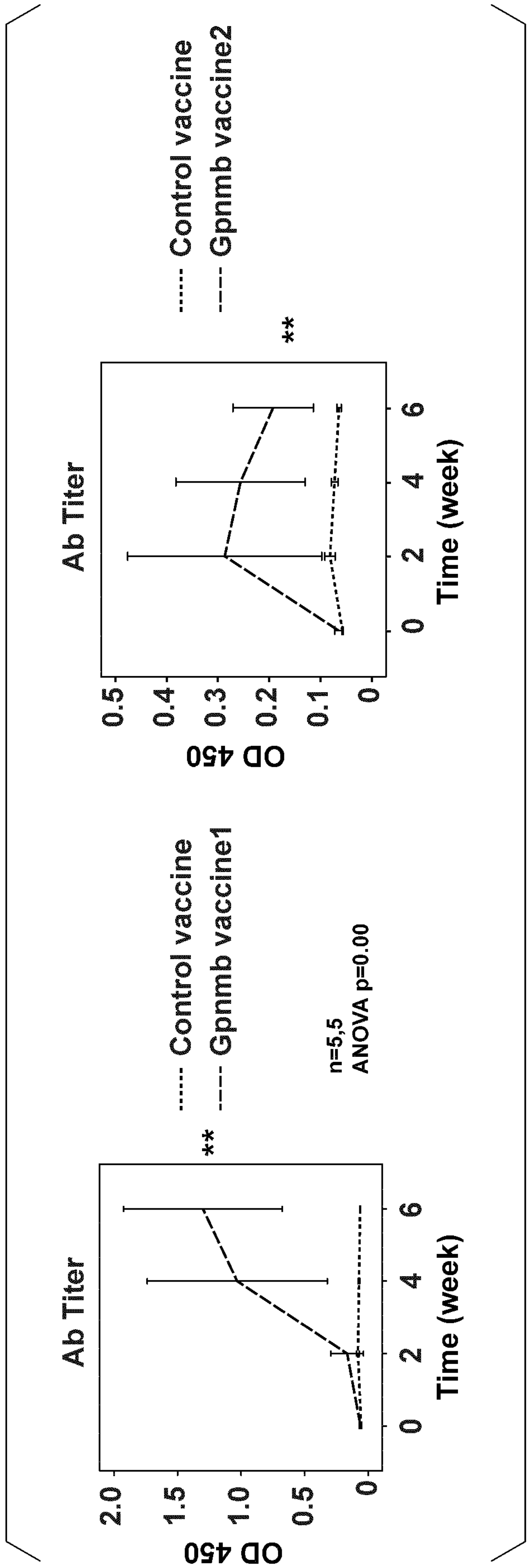
Ab Titer
OD 450
Time (week)
Control vaccine
Gpnmb vaccine1
n=5,5
ANOVA p=0.00
**
Ab Titer
OD 450
Time (week)
Control vaccine
Gpnmb vaccine2
**

FIG. 2B

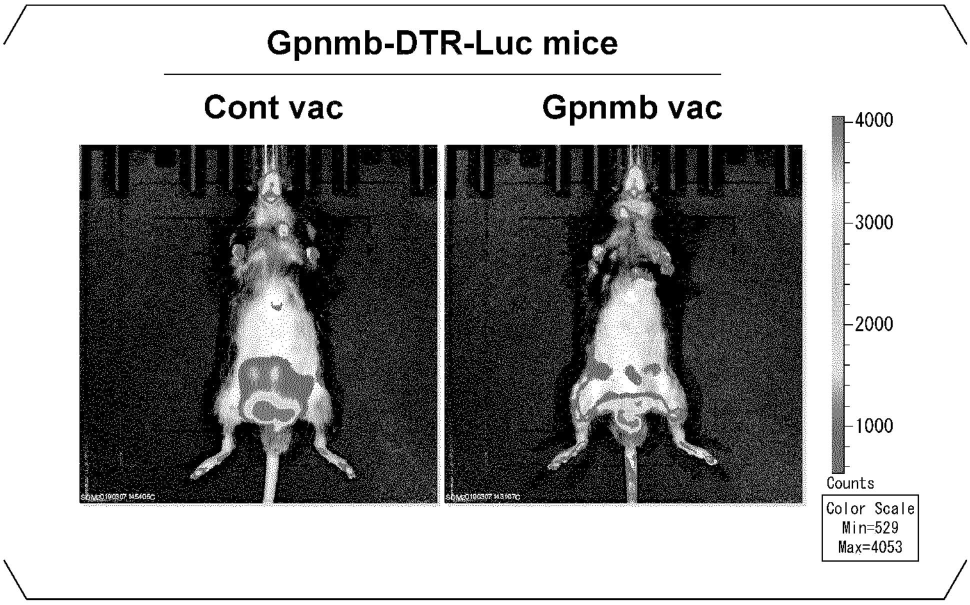
Gpnmb-DTR-Luc mice
Cont vac
Gpnmb vac
4000
3000
2000
1000
Counts
Color Scale
Min=529
Max=4053

FIG. 2C

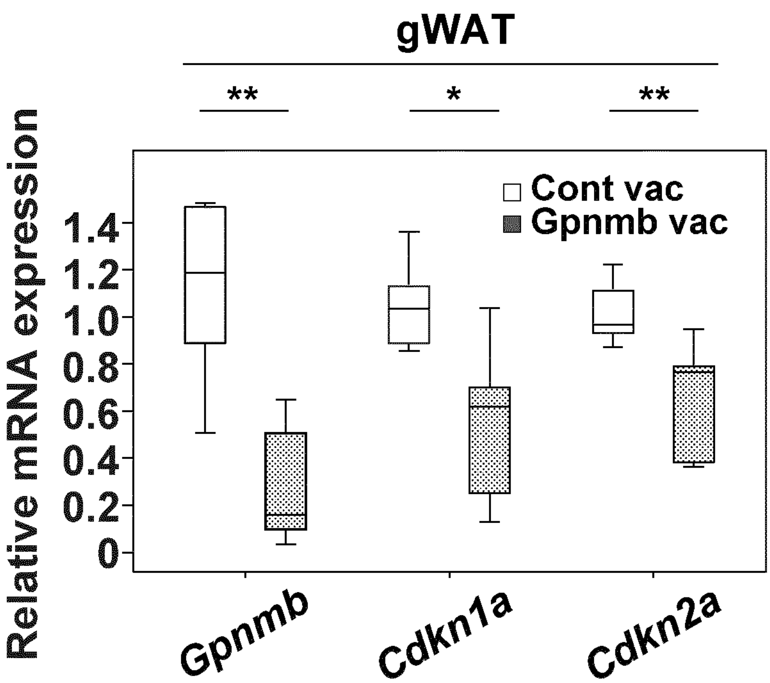
gWAT
**
*
**
Cont vac
Gpnmb vac
Relative mRNA expression
1.4
1.2
1.0
0.8
0.6
0.4
0.2
0
Gpnmb
Cdkn1a
Cdkn2a

FIG. 2D

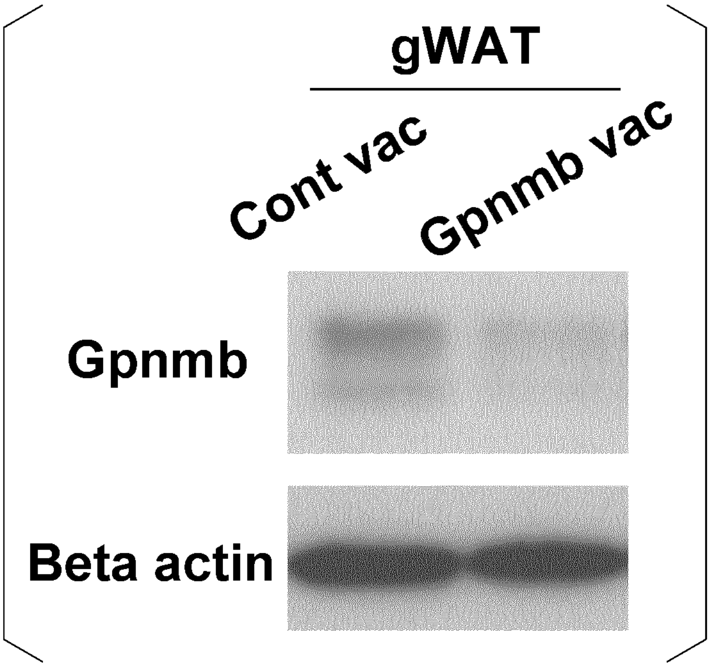
gWAT
Cont vac
Gpnmb vac
Gpnmb
Beta actin

FIG. 2E

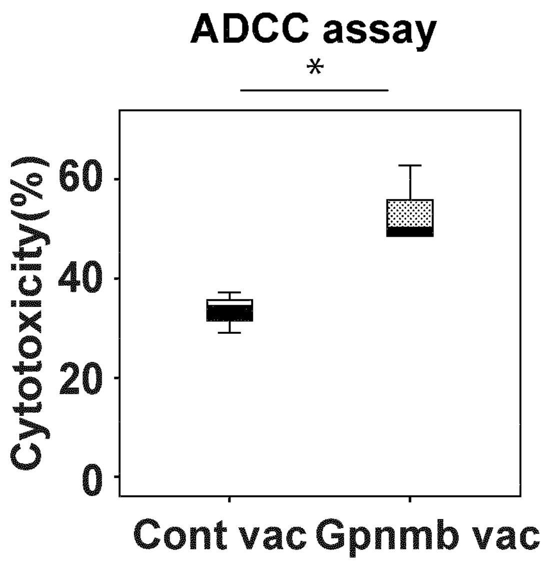
ADCC assay
*
Cytotoxicity(%)
60
40
20
0
Cont vac
Gpnmb vac

FIG. 2F

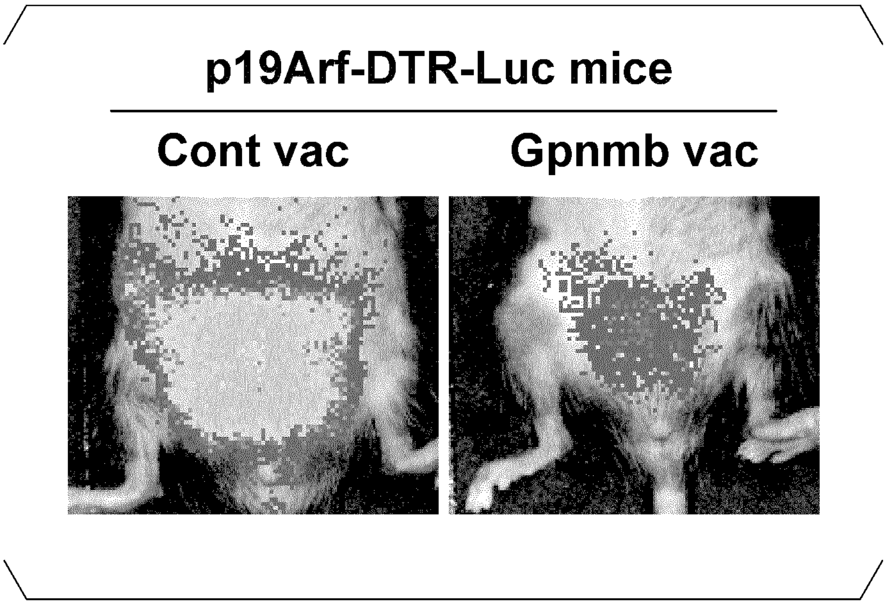


FIG. 2G

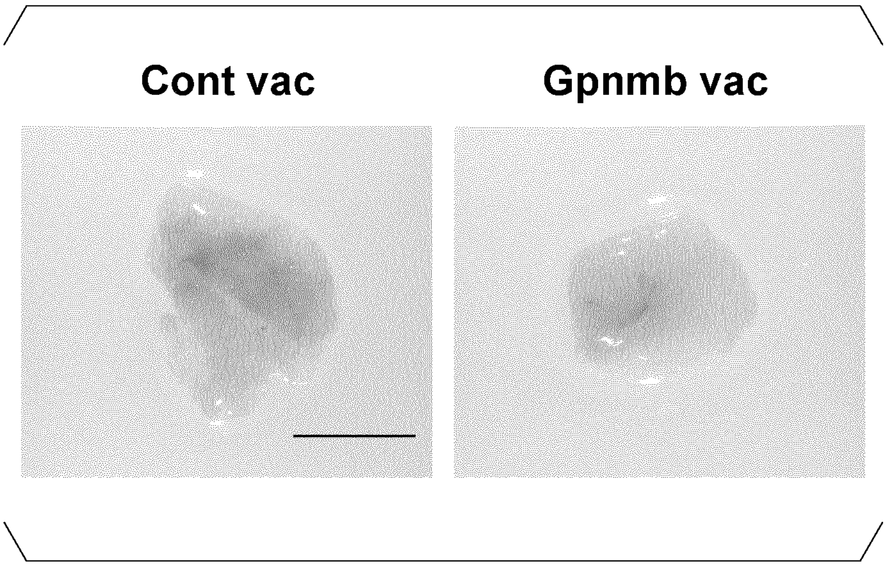

FIG. 2H

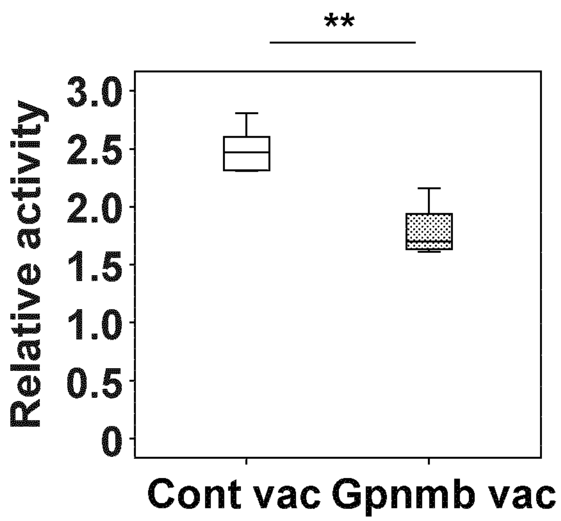

FIG. 2I

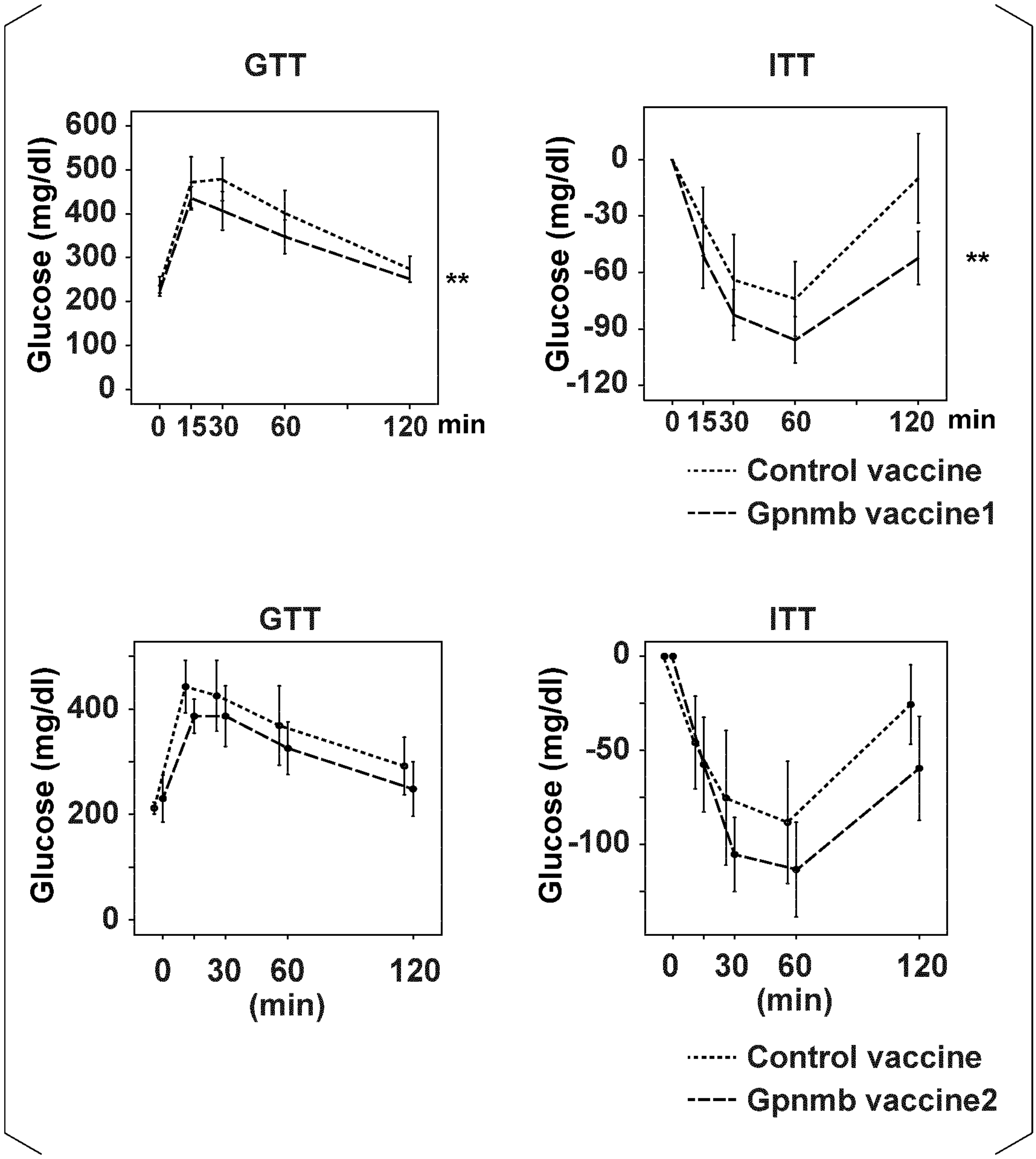
GTT
Glucose (mg/dl)
600
500
400
300
200
100
0
0 1530 60 120 min
**
ITT
Glucose (mg/dl)
0
-30
-60
-90
-120
0 1530 60 120 min
**
Control vaccine
Gpnmb vaccine1
GTT
Glucose (mg/dl)
400
200
0
0 30 60 120
(min)
ITT
Glucose (mg/dl)
0
-50
-100
0 30 60 120
(min)
Control vaccine
Gpnmb vaccine2

FIG. 2J

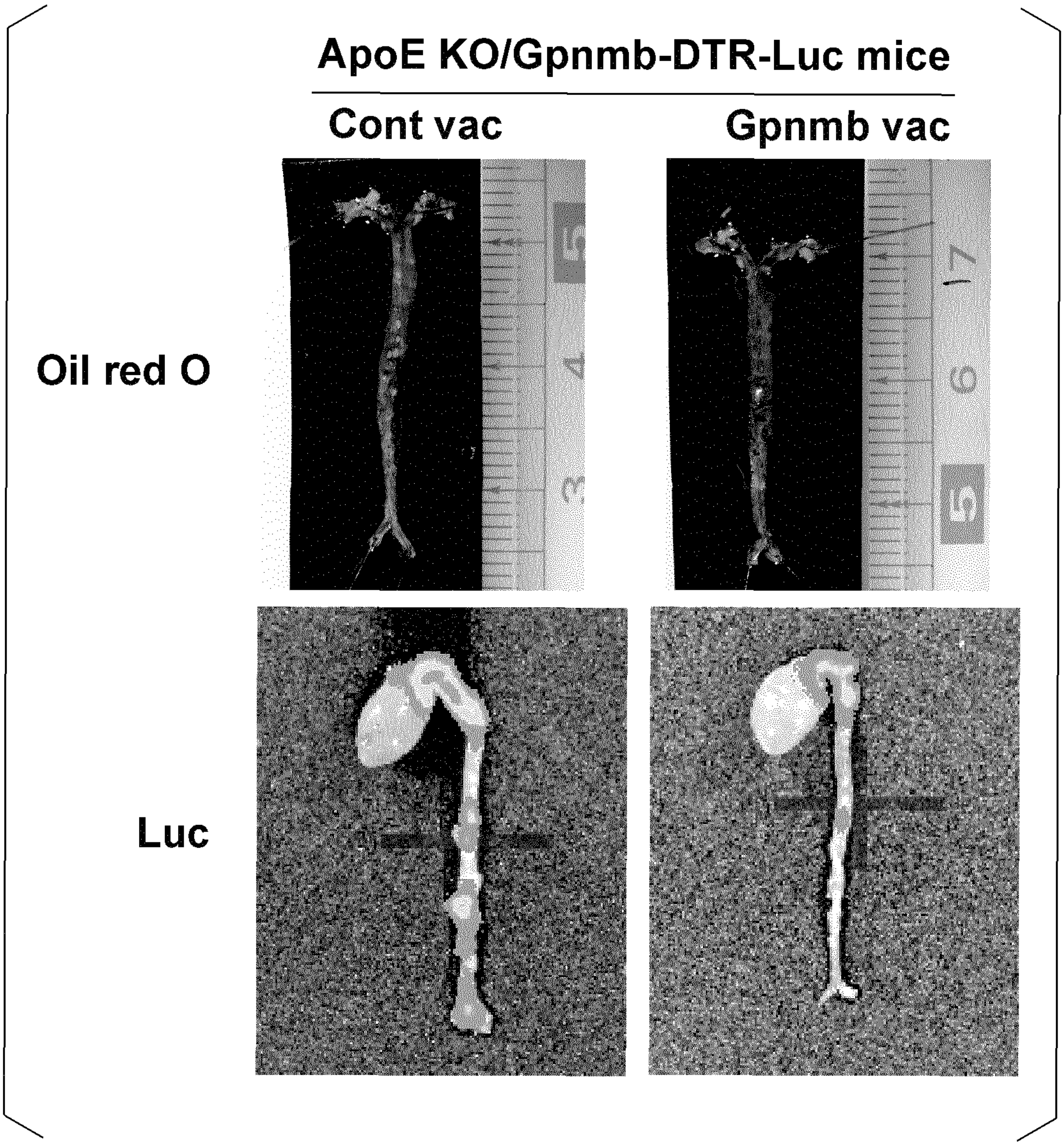

FIG. 2K

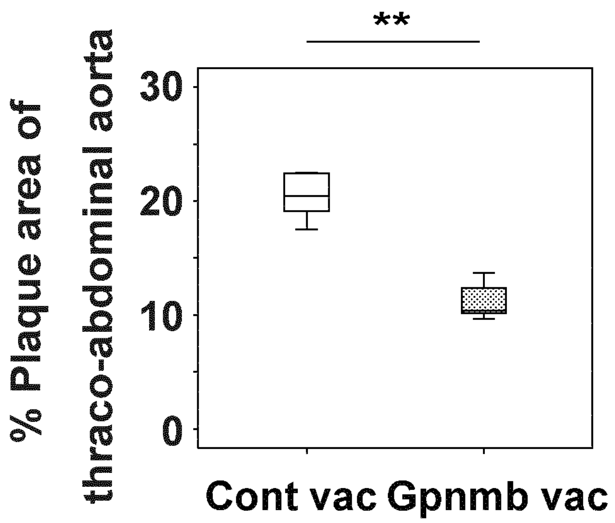
**
% Plaque area of thraco-abdominal aorta
30
20
10
0
Cont vac
Gpnmb vac

FIG. 2L

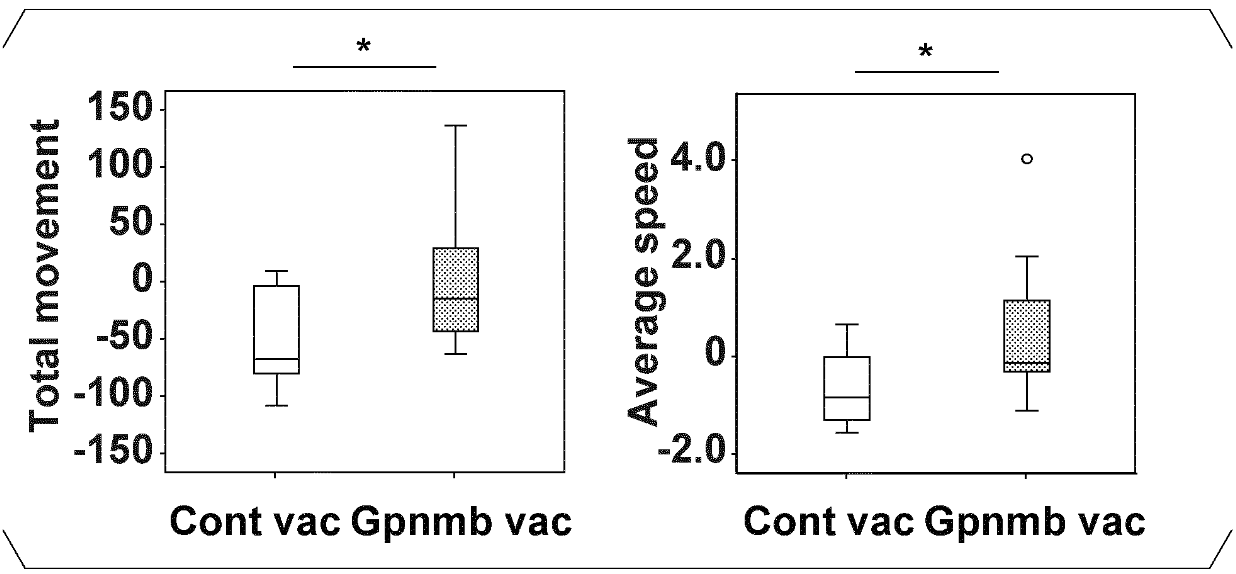
*
Total movement
150
100
50
0
-50
-100
-150
Cont vac
Gpnmb vac
*
Average speed
4.0
2.0
0
-2.0
Cont vac
Gpnmb vac

FIG. 2M

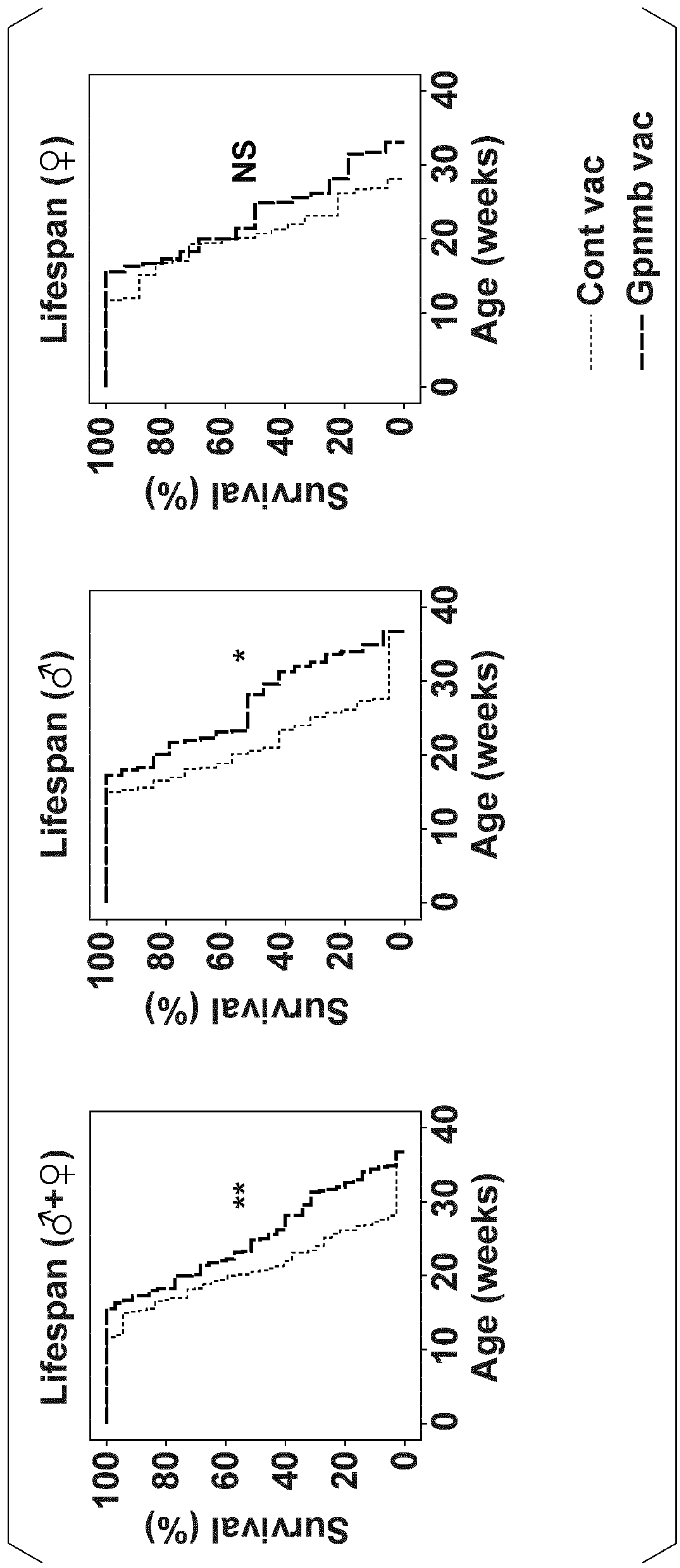

Lifespan (♂+♀)
**
Lifespan (♂)
*
Lifespan (♀)
NS
Survival (%)
100
80
60
40
20
0
Age (weeks)
0
10
20
30
40
Cont vac
Gpnmb vac

## INTERNATIONAL SEARCH REPORT

International application No.
PCT/JP2020/026560

A. CLASSIFICATION OF SUBJECT MATTER
A61K 39/00(2006.01)i; A61K 31/7088(2006.01)i; A61P 37/04(2006.01)i; C07K 7/00(2006.01)n
FI: A61K39/00 H ZNA; A61P37/04; A61K31/7088; C07K7/00

According to International Patent Classification (IPC) or to both national classification and IPC

B. FIELDS SEARCHED

Minimum documentation searched (classification system followed by classification symbols)
A61K39/00; A61K31/7088; A61P37/04; C07K7/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | JP 2017-112849 A (NIIGATA UNIVERSITY) 29.06.2017 (2017-06-29) claim 1 | 1-6 |
| Y | JP 2019-518763 A (SIWA CORPORATION) 04.07.2019 (2019-07-04) claim 1, paragraphs [0006], [0049] | 1-6 |
| A | WO 2009/054470 A1 (TORAY INDUSTRIES, INC.) 30.04.2009 (2009-04-30) entire text | 1-6 |
| A | WO 2018/043463 A1 (NIIGATA UNIVERSITY) 08.03.2018 (2018-03-08) entire text | 1-6 |
| A | 中神啓徳，アンチエイジングを目指した免疫遺伝子治療，Heart View, 2013, vol. 17, pp. 417-422, entire text, non-official translation (NAKAGAMI, Hironori, "immuno-gene therapy aimed at anti-aging") | 1-6 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 September 2020 (23.09.2020) | 06 October 2020 (06.10.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/026560

| Patent Documents referred in the Report | Publication Date | Patent Family | Publication Date |
|---|---|---|---|
| JP 2017-112849 A | 29 Jun. 2017 | (Family: none) | |
| JP 2019-518763 A | 04 Jul. 2019 | US 2019/0328873 A1<br>WO 2017/222535 A1<br>claim 1, paragraphs [0006], [0047]<br>EP 3475306 A1 | |
| WO 2009/054470 A1 | 30 Apr. 2009 | (Family: none) | |
| WO 2018/043463 A1 | 08 Mar. 2018 | US 2019/0192482 A1<br>entire text<br>EP 3508222 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019139031 A
- JP 2017112849 A
- WO 2009054470 A
- JP 2008521411 A


### Non-patent literature cited in the description


- **HASHIMOTO M et al.** Elimination of p19ARF-expressing cells enhances pulmonary function in mice. *JCI Insight,* 2016, vol. 1 (12), e87732